(19) Europäisches Patentamt
European Patent Office
Office européen des brevets

(11) Numéro de publication: **0 360 637 B1**

(12) # FASCICULE DE BREVET EUROPEEN

(45) Date de publication de fascicule du brevet: **04.05.94**

(21) Numéro de dépôt: **89402300.1**

(22) Date de dépôt: **17.08.89**

(51) Int. Cl.5: **C07D 307/94**, C07D 409/04, A61K 31/34, A61K 31/38, A61K 7/06, A61K 7/42, A61K 7/48

(54) **Nouveaux composés benzofuranniques, leur procédé de préparation, compositions pharmaceutique et cosmétique les contenant et utilisation de ces compositions.**

(30) Priorité: **19.09.88 FR 8812173**

(43) Date de publication de la demande: **28.03.90 Bulletin 90/13**

(45) Mention de la délivrance du brevet: **04.05.94 Bulletin 94/18**

(84) Etats contractants désignés: **BE CH DE GB IT LI NL**

(56) Documents cités:
**GB-A- 2 187 455**
**US-A- 4 456 618**

(73) Titulaire: **L'OREAL**
**14, Rue Royale**
**F-75008 Paris(FR)**

(72) Inventeur: **Lang, Gérard**
**44, avenue Lacour**
**F-95210 Saint Gratien(FR)**
Inventeur: **Forestier, Serge**
**16, allée Ferdinand Buisson**
**F-77410 Claye-Souilly(FR)**
Inventeur: **Lagrange, Alain**
**29, rue Auguste Renoir**
**F-78400 Chatou(FR)**
Inventeur: **Maignan, Jean**
**8, rue Halévy**
**F-9230/ Tremblay-les-Gonesse(FR)**
Inventeur: **Malle, Gérard**
**18, Grande-Rue**
**F-77580 Villiers sur Morin(FR)**

(74) Mandataire: **Michardière, Bernard et al**
**Cabinet Peuscet**
**68, rue d'Hauteville**
**F-75010 Paris (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 360 637 B1

## Description

L'invention concerne, à titre de produits nouveaux, des dérivés benzofuranniques, ainsi que les procédés de préparation de ces produits. L'invention concerne également l'utilisation de ces nouveaux composés dans des compositions cosmétiques et des compositions pharmaceutiques utilisables soit en médecine humaine, soit en médecine vétérinaire. Ces composés ont une action de type rétinoïde, c'est-à-dire analogue à celle de la vitamine A.

L'action thérapeutique de la vitamine A sous sa forme acide, aldéhyde ou alcool, est bien connue en dermatologie (voir à cet égard la publication "EXPERIENTIA", volume 34, pages 1105-1119 (1978)). Cette action dans le traitement des proliférations cutanées, de l'acné, du psoriasis et des affections analogues, sera désignée ci-après par l'expression générique "action de type rétinoïde". On a constaté que certains produits ayant une structure analogue à la vitamine A présentaient également une action de type rétinoïde. De plus, certains de ces composés présentent cette action de façon nettement plus élevée que la vitamine A sans que l'effet secondaire d'hypervitaminose toxique soit augmenté proportionnellement (voir à cet égard EUR. J. MED. CHEM. -CHIMICA/THERAPEUTICA Janvier-Février 1980, 15 n° 1, pages 9-15).

Dans le FR-A 2 596 050 du 13 Février 1987, la société déposante a déjà décrit des dérivés benzofuranniques, c'est-à-dire comportant un noyau de 2,3,4,4a-tétrahydro-4a, 10,10-triméthyl-1H-3,9b méthano-dibenzofuranne appelé par la suite TTMDBF, de formule :

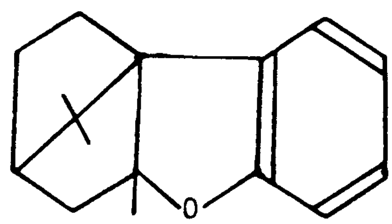

Dans ce brevet, on décrit des composés benzofuranniques ayant une action de type rétinoïde dont la formule générale est :

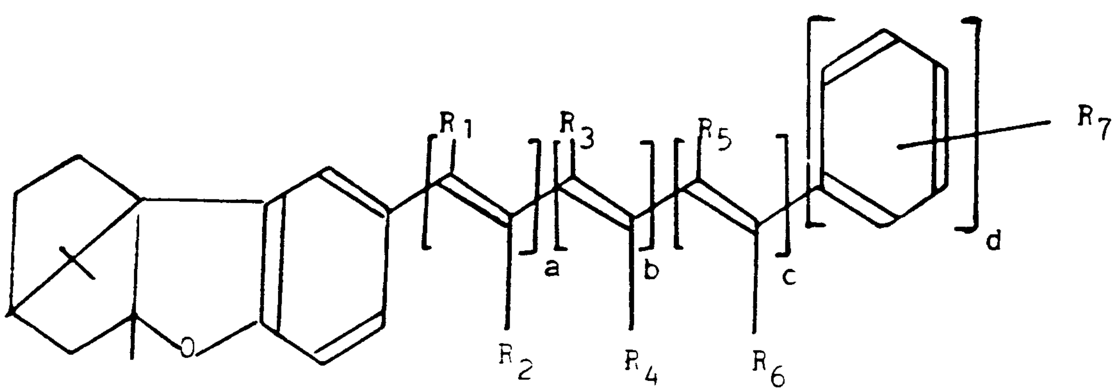

formule dans laquelle :

- a, b, c et d sont des entiers pouvant prendre, indépendamment l'un de l'autre, les valeurs 0 ou 1, avec la condition que la somme a + b + c + d soit supérieure ou égale à 2 ;
- $R_1$, $R_2$, $R_3$, $R_4$, $R_5$ et $R_6$ représentent, indépendamment, un atome d'hydrogène, un radical alkyle en $C_1$-$C_6$ ;
- $R_7$ représente :
  . un radical correspondant à la formule : $-CH_2OR_8$
  formule dans laquelle $R_8$ représente un atome d'hydrogène, un radical alkyle en $C_1$-$C_6$, un radical mono-ou polyhydroxyalkyle en $C_2$-$C_6$,

. un radical correspondant à la formule :

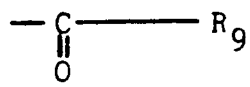

formule dans laquelle $R_9$ représente :
. un atome d'hydrogène ;
. un radical alkyle en $C_1$-$C_6$ ;
. un radical

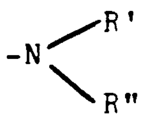

,

où R′ et R″, identiques ou différents, représentent un atome d'hydrogène, un radical alkyle en $C_1$-$C_6$, un radical mono-ou polyhydroxyalkyle en $C_2$-$C_6$, un radical alkényle en $C_3$-$C_6$, R′ et R″ pouvant former un hétérocycle avec l'atome d'azote auquel ils se rattachent, le radical

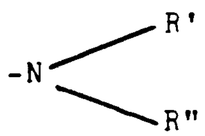

pouvant, en outre, être le reste d'un aminoacide ou d'un amino-sucre ;
. un radical $-OR_{10}$, où $R_{10}$ représente un atome d'hydrogène, un radical alkyle en $C_1$-$C_{20}$, un radical mono-ou polyhydroxyalkyle en $C_2$-$C_6$, le groupement $-OR_{10}$ pouvant être dérivé d'un sucre ;

ainsi que les sels et isomères de ce composé chimique.

La présente demande concerne de nouveaux dérivés benzofuranniques à action de type rétinoïde. Ces nouveaux dérivés benzofuranniques peuvent être utilisés dans des préparations pharmaceutiques pour le traitement des affections dermatologiques liées à un désordre de la kératinisation (différenciation-prolifération), pour le traitement des affections dermatologiques, ou autres, à composante inflammatoire et/ou immuno-allergique, pour le traitement de l'atopie, qu'elle soit cutanée ou respiratoire, pour le traitement des maladies de dégénérescence du tissu conjontif et des tumeurs, pour le traitement du rhumatisme psoriasique, ou pour le traitement de maladies du domaine ophtalmologique, notamment pour le traitement des cornéopathies.

On peut également les utiliser dans des compositions cosmétiques pour l'hygiène corporelle et capillaire.

La présente invention concerne les composés benzofuranniques ayant pour formule générale (I) :

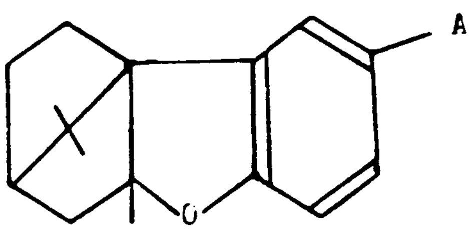

(I)

dans laquelle A représente :

- soit un groupement (II) :

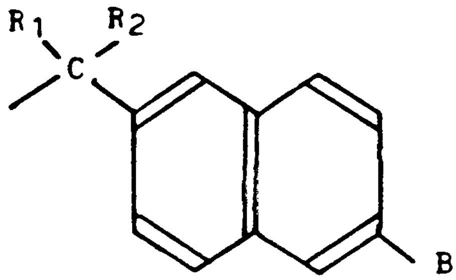

(II)

- soit un groupement (III) :

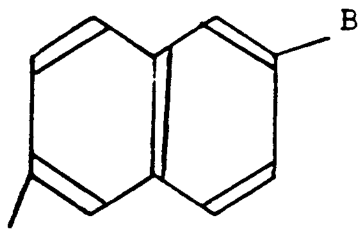

(III)

- soit un groupement (IV) :

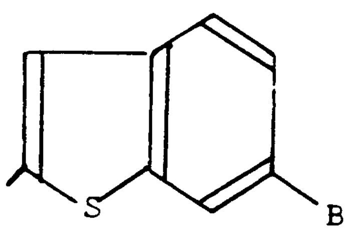

(IV)

- soit un groupement (V) :

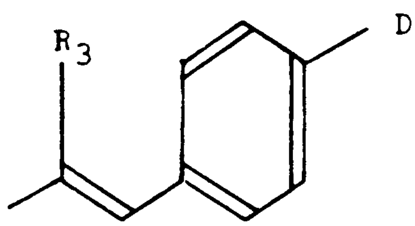

(V)

dans le groupement (II), $R_1$ représentant un atome d'hydrogène, un radical OH, un radical alcoxy en $C_1$-$C_4$, un radical acyloxy en $C_1$-$C_4$, ou un radical $NH_2$ ; $R_2$ représentant un atome d'hydrogène, ou un radical alcoxy en $C_1$-$C_4$; ou bien $R_1$ et $R_2$ formant, pris ensemble, un radical oxo, méthano ou hydroxyimino ; dans les groupements (II) à (IV), B représentant :

- un radical CN ;
- un radical oxazolinyle ;
- un radical correspondant à la formule :

$-CH_2OR_4$

formule dans laquelle $R_4$ représente un atome d'hydrogène, un radical alkyle en $C_1$-$C_6$, un radical mono- ou polyhydroxyalkyle en $C_2$-$C_6$, un radical cyclopentyle, un radical cyclohexyle ou un radical tétrahydropyrannyle ;

- un radical correspondant à la formule

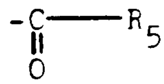

dans laquelle $R_5$ représente :

. un atome d'hydrogène ;
. un radical alkyle en $C_1$-$C_6$ ;
. un radical

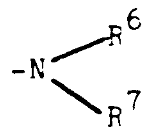

où $R^6$ et $R^7$ représentent un atome d'hydrogène, un radical alkyle en $C_1$-$C_6$, un radical alcényle en $C_3$-$C_6$, un radical cyclopentyle ou cyclohexyle ou encore un radical aralkyle ou aryle éventuellement substitués, $R^6$ et $R^7$ pouvant former un cycle avec l'atome d'azote auquel ils se rattachent, le radical

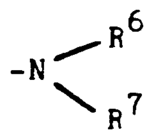

pouvant, en outre, être le reste d'un aminoacide ou d'un amino-sucre ;

. un radical -O-$R_8$, où $R_8$ représente un atome d'hydrogène, un radical alkyle en $C_1$-$C_{20}$ , un reste mono-ou polyhydroxyalkyle en $C_2$-$C_6$, le groupement -O-$R_8$ pouvant également être dérivé d'un sucre ;

- un radical D représentant soit un reste -SH, soit un reste -$S(O)_nR_9$ dans lequel n est compris entre 0 et 2, bornes incluses, $R_9$ représentant :

. un radical alkyle en $C_1$-$C_6$ substitué ou non par un ou plusieurs groupes alcoxycarbonyle en $C_2$-$C_6$, un ou plusieurs groupements carboxyles, un ou plusieurs groupes amino ou dialkylamino ou pouvant être un reste d'un aminoacide ;
. un radical alcényle en $C_3$-$C_6$ ;
. un radical mono- ou polyhydroxyalcoyle en $C_2$-$C_6$ ;
. et, lorsque n est égal à 1 ou 2, un reste hydroxy, un reste alcoxy en $C_1$-$C_6$ ou un reste

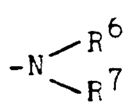

dans lequel $R^6$ et $R^7$ peuvent avoir les significations définies ci-dessus ;

dans le groupement (V), D ayant la signification donnée ci-dessus et $R_3$ représentant un reste alkyle en $C_1$-$C_6$,

ainsi que les sels et isomères de ces composés.

La présente invention concerne plus particulièrement les composés de formule (I) dans laquelle A est un groupement (II) où B est soit $COR_5$ avec

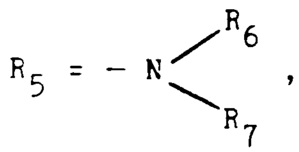
,

$R_6$ étant H et $R_7$ étant un radical alkyle en $C_1$-$C_6$, soit $COOR_8$ avec $R_8$ = H ou un radical alkyle en $C_1$-$C_{20}$.

La présente invention se rapporte plus particulièrement aussi aux composés de formule (I) dans laquelle A est un groupement (III) où B est soit $COR_5$ avec

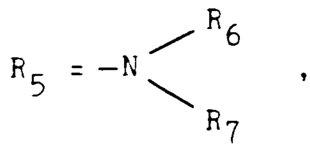
,

$R_6$ étant H et $R_7$ étant un radical alkyle en $C_1$-$C_6$ soit $COOR_8$ avec $R_8$ = H ou un radical alkyle en $C_1$-$C_{20}$.

Les composés de formule (I) où A est un groupement (IV) sont, de préférence, ceux où B est $COOR_8$, $R_8$ étant H ou un radical alkyle en $C_1$-$C_{20}$.

Les composés de formule (I) où A est un groupement (V) sont, de préférence, ceux où D est $-S-CH_3$.

La présente invention a également pour objet les procédés de préparation des composés benzofuranniques définis ci-dessus.

Les composés de formule (I) dans lesquels A est le groupement (II) sont préparés de la façon suivante : on prépare le plus souvent d'abord le composé dans lequel $R_1$ et $R_2$ forment ensemble un radical oxo et B représente un radical $CO_2R_8$, $R_8$ étant un radical alkyle en $C_1$-$C_{20}$. Ce produit sert ensuite de produit de départ pour la fabrication d'autres composés.

Ce produit de départ est préparé de la façon suivante : en premier lieu, le produit (1), à savoir l'acide alcoxycarbonyl-6 naphtalène carboxylique-2 est transformé en chlorure correspondant par action du chlorure de thionyle, selon le schéma réactionnel suivant :

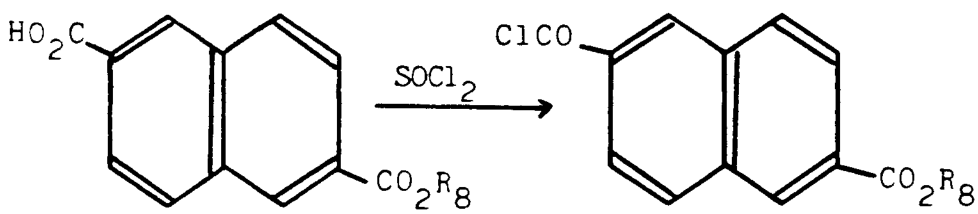


1 2

en second lieu, on fait réagir ensuite le chlorure d'acide obtenu (2) avec le 2,3,4,4a-tétrahydro-4a,10,10-triméthyl-1H-3,9b méthano-dibenzofuranne (3) soit directement par réaction de Friedel et Crafts, soit par réaction sur un dérivé magnésien halogéné (4) préalablement préparé, selon le schéma réactionnel suivant :

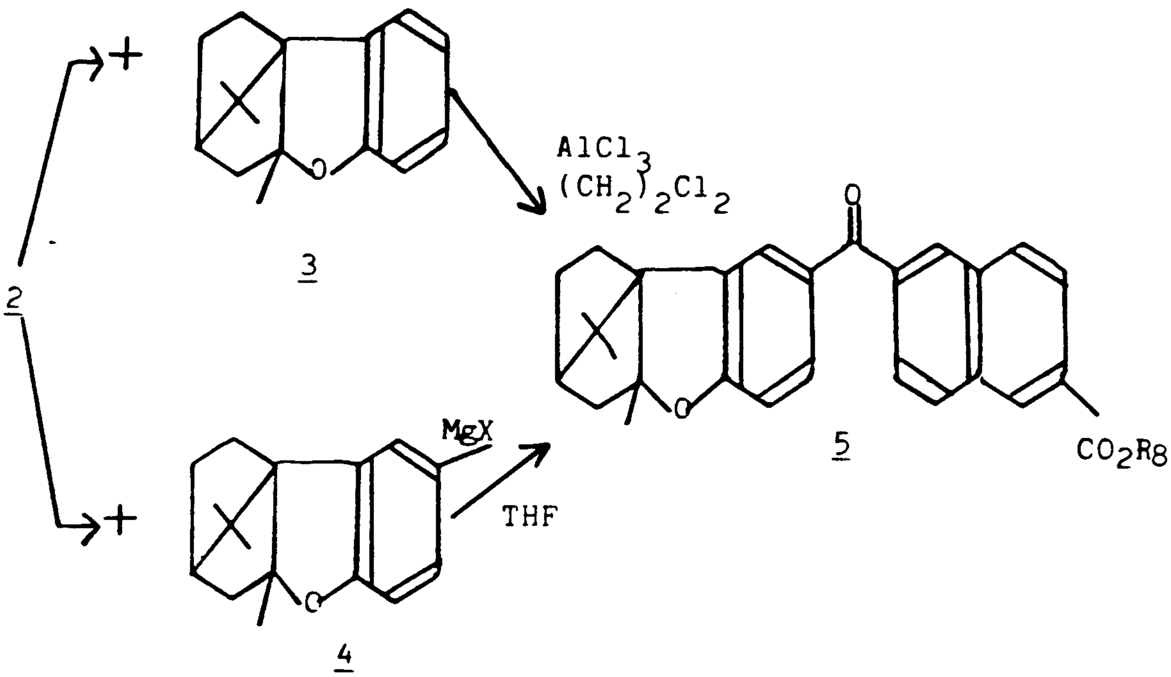


2 3 4 5

X représentant Br ou Cl, l'ester benzofurannique obtenu (5) pouvant être ensuite saponifié pour donner le cétoacide (6) selon le schéma réactionnel suivant :

5

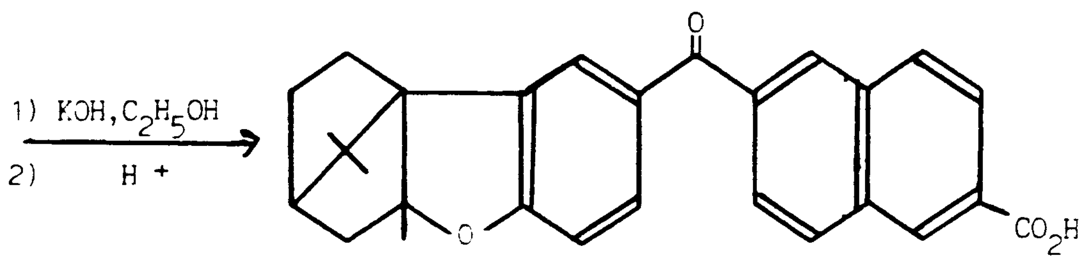


6

l'ester benzofurannique (5) ou l'acide benzofurannique (6) obtenus servant ensuite de façon connue de produits de départ pour la fabrication d'autres dérivés de formule (I) dans laquelle A est le groupement (II).

Le produit (1) à savoir l'acide alcoxycarbonyl-6 naphtalène carboxylique-2 peut, par exemple, être obtenu par réaction de monosaponification d'un naphtalène dicarboxylate d'alkyle-2,6, de préférence à partir du naphtalène dicarboxylate de méthyle-2,6 qui est un produit commercial.

Le dérivé magnésien (4) peut être préparé dans le tétrahydrofuranne (THF) anhydre au reflux et la condensation du chlorure d'acide (2) est réalisée à une température de 0 ° C dans le même solvant.

On peut, par exemple, ensuite préparer un amide (7) à partir du cétoacide (6) selon le schéma suivant :

6

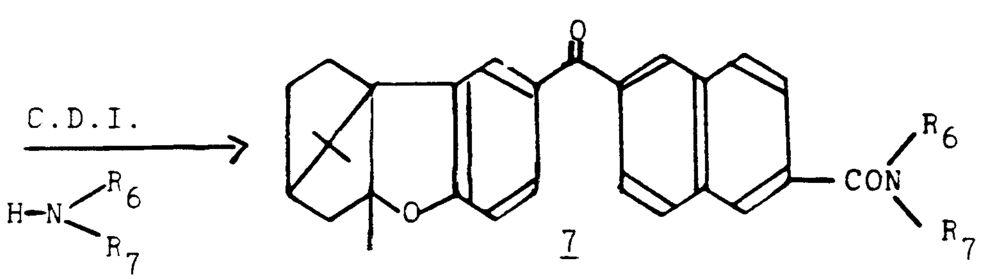


7

par action d'une amine de formule

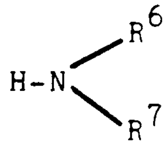

en présence de N,N'-carbonyldiimidazole (CDI).

Pour certaines significations de $R_8$, en particulier lorsque $R_8$ représente un radical monohydroxy- ou polyhydroxyalkyle, il est préférable de préparer le cétoacide (6) à partir de l'ester méthylique (5) dans lequel $R_8$ est $CH_3$ et ensuite d'estérifier l'acide ainsi obtenu en ester de l'alcool choisi selon des méthodes connues.

Le composé de formule (I) dans laquelle A représente le groupement (II), $R_1$ = H, $R_2$ = OH et B = $CH_2OH$ est obtenu par réduction du cétoacide (6) en présence d'hydrure de lithium aluminium dans le tétrahydrofuranne (T.H.F.) selon le schéma réactionnel suivant :

6

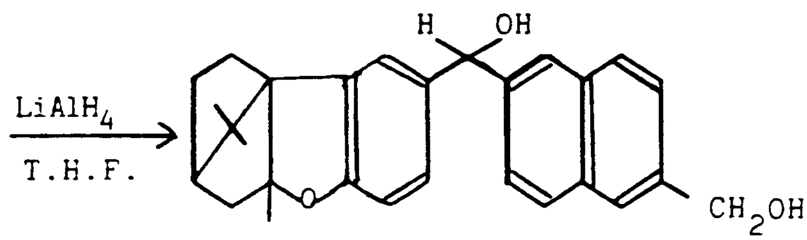


8

Le diol obtenu (8) peut alors être oxydé en présence de chlorochromate de pyridinium (P.C.C.) pour conduire au céto-aldéhyde (9) selon le schéma réactionnel suivant :

8

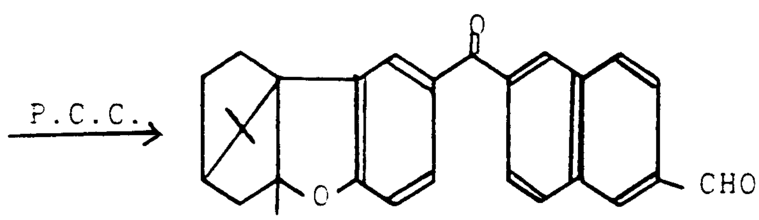


9

Les composés de formule (I) dans laquelle A représente le groupement (II), $R_1$ = H, $R_2$ = OH et B = $CO_2R_8$ sont obtenus à partir des dérivés cétoniques par réduction au borohydrure de sodium dans le tétrahydrofuranne.

Les composés de formule (I) dans laquelle A représente le groupement (II), et $R_1$ et $R_2$ sont H, sont obtenus par réduction au zinc des dérivés cétoniques correspondants dans l'acide acétique, en présence d'acide chlorhydrique. Ces réactions de réduction du carbonyle doivent, bien entendu, être compatibles avec la nature du radical B. La réduction du carbonyle ne soulève aucune difficulté lorsque B = $CO_2H$, mais il peut être souhaitable pour d'autres radicaux B d'en assurer éventuellement la protection.

Les composés acyloxy de formule (I) dans laquelle A représente le groupement (II), avec $R_2$ = H et $R_1$ = acyloxy en $C_1$-$C_4$,sont obtenus en faisant réagir un anhydride ou un chlorure d'acide sur un composé de formule (I) dans laquelle A représente le groupement (II) et $R_2$ = H, $R_1$ = OH.

Les composés alcoxy de formule (I) dans laquelle A représente le groupement (II), avec $R_1$ = H et $R_2$ = alkoxy en $C_1$-$C_4$, sont de même obtenus à partir des composés correspondants pour lesquels $R_1$ = H et $R_2$ = OH, selon les méthodes connues d'éthérification.

Pour la préparation des dérivés acyloxy et alkoxy, il est préférable que le radical B soit une fonction ester ou acide.

Les composés de formule (I) dans laquelle A représente le groupement (III) sont obtenus par réaction de couplage entre un composé benzofurannique halogéné de formule (10) et un dérivé halogéné du naphtalène de formule (11) :

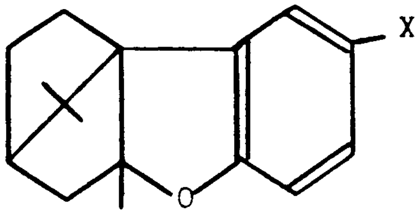

10

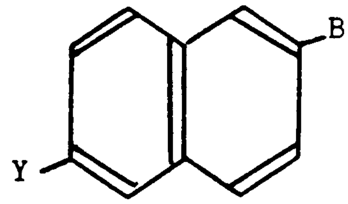

11

dans lesquelles B a la même signification que celle donnée ci-dessus et X et Y représentent un atome d'halogène, le composé benzofurannique halogéné de formule (10) étant transformé en son dérivé magnésien, lithien ou zincique puis couplé avec le dérivé halogéné du naphtalène de formule (11) en utilisant, comme catalyseur de réaction, un métal de transition ou l'un de ses complexes. Comme catalyseur, on peut, en particulier, mentionner ceux dérivés du nickel, du palladium et, en particulier, les composés de $Ni_{II}$, par exemple $NiCl_2$, avec différentes phosphines. La réaction de couplage est généralement effectuée à une température comprise entre -20 °C et +30 °C dans un solvant anhydre, tel que par exemple le diméthylformamide ou le tétrahydrofuranne. Le produit obtenu peut être purifié par recristallisation ou par chromatographie sur colonne de silice. Il va de soi que le choix du dérivé halogéné du naphtalène de formule (11), pour la réaction de couplage avec le composé halogéné de formule (10), doit être tel qu'il puisse conduire par réaction ultérieure aux différentes significations du radical B définies ci-dessus.

On prépare les composés de formule (I) dans laquelle A est un groupement (IV) en condensant un dérivé benzofurannique d'acide carboxylique (12) avec un thiol (13),selon la réaction :

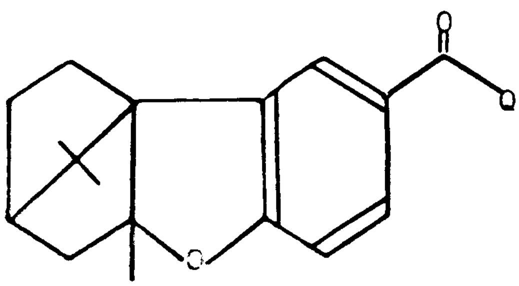

12

+

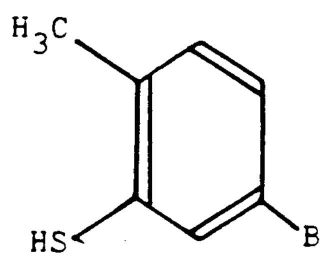

13

le radical Q étant OH ou Cl.

On obtient un produit intermédiaire (14) de formule :

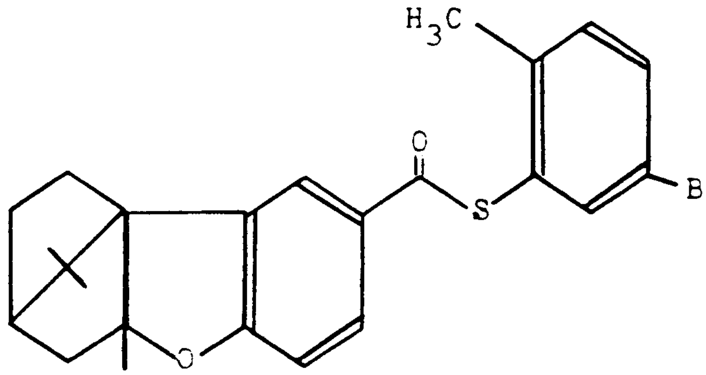

14

qui est soumis ensuite à une bromation pour obtenir un dérivé bromométhylé (15) de formule :

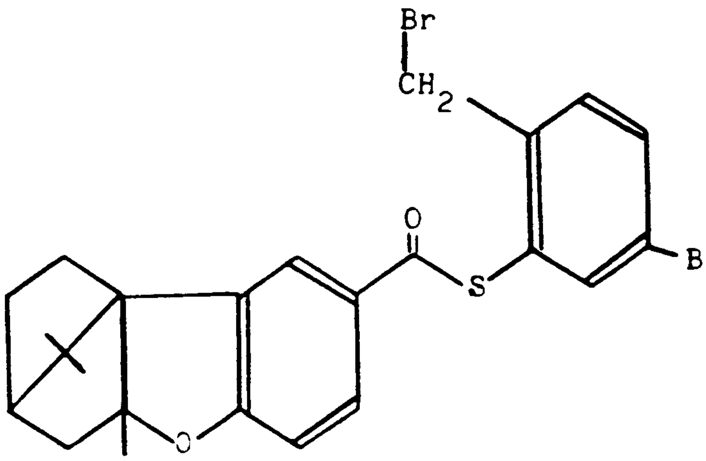

15

Le produit (15) est ensuite soumis à une réaction avec une triarylphosphine puis à une cyclisation pour obtenir le produit (16) de formule :

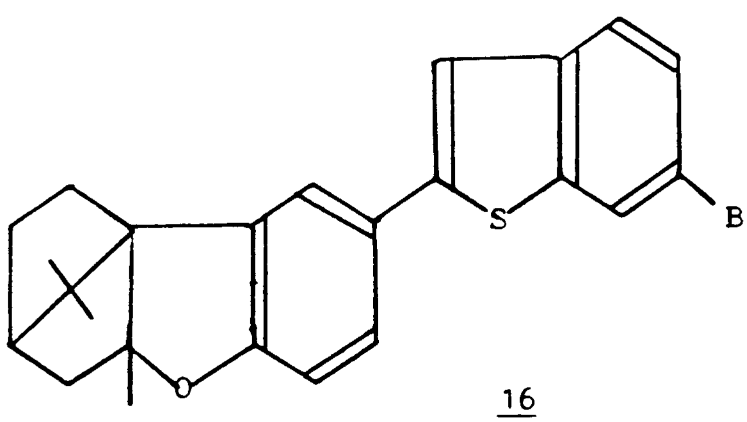

16

La réaction de bromation est réalisée, de préférence, au moyen de N-bromosuccinimide dans le benzène ou le tétrachlorure de carbone sec à une température comprise entre 70 °C et 90 °C en présence

d'un initiateur radicalaire tel que le peroxyde de benzoyle, éventuellement sous irradiation lumineuse.

La réaction de cyclisation est, de préférence, réalisée en présence d'une base telle qu'un hydroxyde de métal alcalin ou un carbonate de métal alcalin, par exemple l'hydroxyde de lithium ou le carbonate de potassium, d'un hydrure de métal alcalin, par exemple l'hydrure de sodium, d'un alcoolate de métal alcalin, par exemple le méthylate de sodium ou le tertio-butylate de potassium, d'une amine tertiaire, par exemple la triéthylamine, la diisopropyléthylamine ou le diazabicyclo undécène, d'un amidure de métal alcalin, par exemple l'amidure de sodium ou le diisopropylamidure de lithium. La température de réaction est comprise entre -10 °C et +150 °C. On utilise, de préférence, un solvant aprotique dipolaire (diméthyl sulfoxyde ou diméthylformamide), un alcool ou un éther (dioxanne ou tétrahydrofuranne).

Les composés de formule (I) dans laquelle A représente le groupement (V) sont préparés selon l'un des trois procédés suivants :

1) Premier procédé :

On fait réagir un composé benzofurannique de formule :

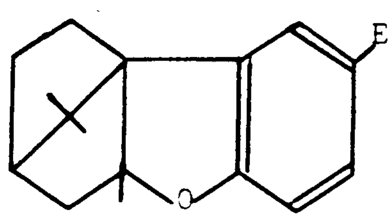

17

avec un composé de formule :

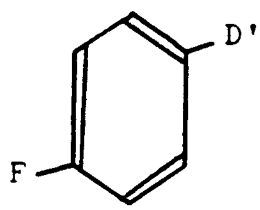

18

formules dans lesquelles D' représente un reste $-SO_3^{\ominus}\ M^{\oplus}$, $-SO_2^{\ominus}\ M^{\oplus}$, alkylthio, alkylsulfinyle ou alkylsulfonyle, dans lesquels le radical alkyle est en $C_1$-$C_6$, E représentant un reste

$$R_3-\overset{|}{C}H-P\,[Z]_3^{\oplus}\ Y^{\ominus}$$

lorsque F représente un reste formyle, ou bien représentant un reste

$$-\overset{\overset{\displaystyle O}{||}}{C}-R_3$$

lorsque F représente un reste

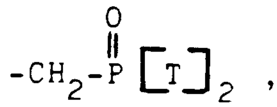

Z étant un reste aryle, T étant un reste alcoxy en $C_1$-$C_6$, $Y^{\ominus}$ étant, un anion d'un acide mineral ou organique et $M^{\oplus}$ étant un cation d'un métal alcalin ou alcalino-terreux, $R_3$ ayant la signification définie ci-dessus.

2) Deuxième procédé :

On fait réagir une base forte sur le composé de formule :

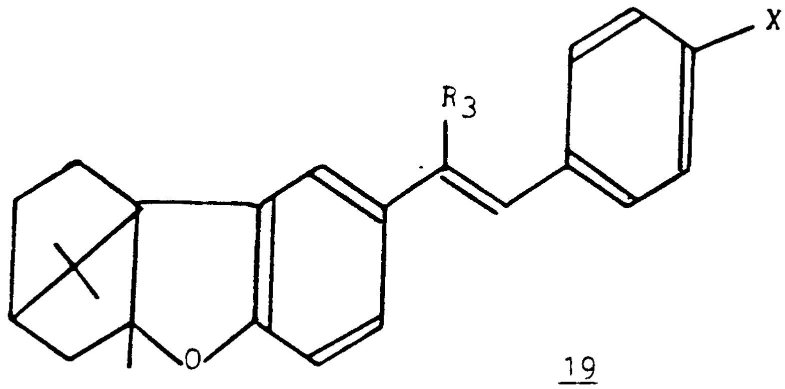

19

dans lequel $R_3$ a la signification définie ci-dessus et X représente un atome d'halogène, puis on soumet le produit obtenu à l'action de $SO_2$.

3) Troisième procédé :

On fait réagir un composé de formule :

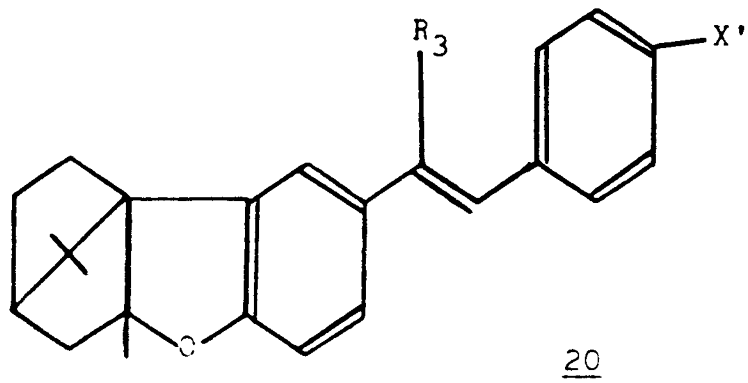

20

dans laquelle $R_3$ a la signification définie ci-dessus et X' représente un reste alkylthiocarbamoyle avec un radical alkyle en $C_1$-$C_6$,
avec une base ou un hydrure métallique.

Le premier procédé peut être mis en oeuvre selon les conditions classiques de la réaction de Wittig ou de la réaction de Horner.

Dans le deuxième procédé, la base forte utilisée est, de préférence, le butyllithium. La réaction est effectuée dans un solvant inerte tel qu'un éther ou un hydrocarbure à une température voisine de 0 °C.

La base utilisée dans le troisième procédé est un hydroxyde de métal alcalin tel que la potasse. La réaction est effectuée dans un solvant inerte à une température comprise entre la température ambiante et le point d'ébullition du solvant. On obtient, selon ce troisième procédé, un composé de formule (I) dans laquelle A représente un groupement (V) avec D = SH.

Dans les composés de formule générale (I), où A est un groupement (II), (III) ou (IV),on peut procéder aux modifications fonctionnelles du reste B ou du reste D selon des méthodes connues :

. acide carboxylique → ester
. ester → acide carboxylique
. acide → chlorure d'acide
. chlorure d'acide → amide
. acide → amide
. acide → alcool
. alcool → aldéhyde
. amide → amine
. thiol → thioéther
. thioéther → sulfoxyde
. thioéther → sulfone
. acide sulfonique → ester sulfonique
. acide sulfonique → sulfonamide
. acide sulfinique → ester sulfinique
. addition de Michael des thiols

De même, la transformation des acides carboxyliques, sulfoniques ou sulfiniques en sels correspondants ou des sels en acides correspondants, peut se faire selon des méthodes connues.

Les composés de formule (I) sont utilisés pour l'obtention d'un médicament destiné à traiter les affections dermatologiques liées à un désordre de la kératinisation portant sur la différenciation et sur la prolifération, notamment pour traiter les acnés vulgaires, comédoniennes, polymorphes, les acnés nodulo kystiques, conglobata, les acnés séniles, les acnés secondaires telles que l'acné solaire, médicamenteuse, professionnelle. Ces composés sont également indiqués pour les autres types de trouble de la kératinisation, notamment les ichtyoses, les états ichtyosiformes, la maladie de Darier, les kératodermies palmoplantaires, les leucoplasies et états leucoplasiformes, le lichen.

Les composés sont également utilisés pour l'obtention d'un médicament destiné à traiter les autres affections dermatologiques liées à un trouble de la kératinisation avec une composante inflammatoire et/ou immuno-allergique et, notamment, toutes les formes de psoriasis,qu'il soit cutané, muqueux ou unguéal et même le rhumatisme psoriasique ; ils peuvent également être utilisés dans le traitement de l'atopie cutanée telle que l'eczema ou de l'atopie respiratoire.

Ces composés peuvent également être utilisés pour l'obtention d'un médicament destiné à traiter toutes les proliférations dermiques ou épidermiques, qu'elles soient bénignes ou malignes, qu'elles soient d'origine virale telles que les verrues vulgaires, les verrues planes et l'épidermodysplasie verruciforme ; les proliférations peuvent également être induites par les ultra-violets telles que les épithelioma baso et spino cellulaires.

Ces composés peuvent également être utilisés pour l'obtention d'un médicament destiné à traiter d'autres désordres dermatologiques tels que les maladies bulleuses et les maladies du collagène.

Enfin, ces composés trouvent aussi une application dans le domaine ophtalmologique pour le traitement des cornéopathies.

La présente invention a donc aussi pour objet une nouvelle composition médicamenteuse destinée notamment au traitement des affections susmentionnées, caractérisée par le fait qu'elle comporte, dans un support pharmaceutiquement acceptable, comme substance(s) active(s), au moins un composé de formule (I) et/ou au moins un de ses isomères et/ou au moins un de ses sels.

Lorsque les composés de formule (I) sont utilisés par voie topique, on observe une bonne activité de ceux-ci sur une très grande gamme de dilution ; on peut utiliser, notamment, des concentrations en substance(s) active(s) allant de 0,0005% à 2% en poids. Il est bien entendu possible d'utiliser des concentrations supérieures, lorsque ceci est rendu nécessaire pour une application thérapeutique particulière ; toutefois, les concentrations préférées en principe actif sont comprises entre 0,002 et 1% en poids.

Les compositions topiques se présentent avantageusement sous la forme d'onguents, de gels, de crèmes, de pommades, de poudres, de teintures, de solutions, de suspensions, d'émulsions, de lotions, de sprays, de timbres ou de tampons imbibés. Les composés de formule (I) sont mélangés à des supports inertes non toxiques, généralement liquides ou pâteux, appropriés au traitement par voie topique.

Les substances pharmaceutiquement actives précitées peuvent être utilisées par voie entérale. Par voie orale, on administre lesdites substances actives à raison d'environ 2 µg jusqu'à 2 mg par jour et par kilogramme de poids corporel ; une posologie excessive peut se manifester sous la forme d'une hypervitaminose A reconnaissable à ses symptômes et pouvant faire craindre une toxicité hépatique nécessitant un contrôle biologique de la fonction hépatique. La dose nécessaire peut être administrée en une ou plusieurs prises. Pour l'administration par voie orale, les formes appropriées sont, par exemple, les comprimés, les gélules, les dragées, les sirops, les suspensions, les émulsions, les solutions, les poudres, les granulés ; un mode d'administration préféré consiste à utiliser des gélules contenant de 0,1 mg à environ 1 mg de substance(s) active(s).

Les substances pharmaceutiquement actives de l'invention peuvent être administrées par voie parentérale, sous forme de solutions ou suspensions pour perfusions ou injections intraveineuses ou intramusculaire. Dans ce cas, on administre lesdites substances actives à raison d'environ 2 µg jusqu'à 2 mg par jour et par kg de poids corporel ; un mode d'administration préféré consiste à utiliser des solutions ou suspensions contenant de 0,01 mg à 1 mg environ de substance(s) active(s) par ml.

Lorsque les substances pharmaceutiquement actives de formule (I) sont utilisées par voie oculaire, elles se présentent, avantageusement, sous forme de solutions ou de poudres à diluer pour collyres.

Le support pharmaceutiquement acceptable peut comporter de l'eau, de la gélatine, du lactose, de l'amidon, du talc, de la vaseline, de la gomme arabique, des polyalcoylène-glycols, du stéarate de magnésium. Les comprimés, poudres, dragées, granulés ou gélules peuvent contenir des liants, des charges, des supports pulvérulents. Les solutions, crèmes, suspensions, émulsions ou sirops peuvent contenir des diluants, des solvants, des épaississants.

Les composés de formule (I) ainsi que les sels et les isomères de ces composés trouvent également une application dans le domaine cosmétique, en particulier dans l'hygiène corporelle et capillaire et, notamment, dans le traitement des peaux à tendance acnéique, des peaux physiologiquement sèches, des séborrhées, de la chute des cheveux, pour la repousse des cheveux, pour lutter contre l'aspect gras de la peau ou des cheveux et trouvent aussi une application contre les effets du vieillissement de la peau. Ils ont également un pouvoir préventif et curatif contre les effets néfastes du soleil.

Les composés selon l'invention présentent une excellente activité comédolytique dans le test de la souris rhino décrit par BUNNE and al. dans International Journal of Cosmetic Science 3, 23-28 (1981).

La présente invention a donc aussi pour objet une nouvelle composition cosmétique, caractérisée par le fait qu'elle comporte, dans un support cosmétiquement acceptable, comme substance(s) active(s), au moins un composé de formule (I) et/ou au moins un de ses isomères et/ou au moins un de ses sels ; cette composition peut se présenter sous forme de lotion, gel, crème, mousse, savon, shampooing ou analogues.

La concentration en substance(s) cosmétiquement active(s) est comprise entre 0,0005 et 2% en poids et, de préférence, entre 0,01 et 1 % en poids par rapport au poids total de la composition.

Dans le traitement des désordres susmentionnés, les composés selon l'invention, utilisés dans les compositions ci-dessus définies, agissent en accroissant la production épithéliale folliculaire des cellules non-adhérentes, délogeant ainsi et faisant partir le contenu du comédon acnéique. Ces composés réduisent la taille des glandes sébacées et inhibent partiellement la sécrétion du sébum.

Les compositions selon l'invention peuvent contenir des additifs inertes, ou bien pharmacodynamiquement ou cosmétiquement actifs et, notamment, des agents hydratants, comme la thiamorpholinone et ses dérivés ou l'urée ; des agents anti-séborrhéiques, tels que la S-carboxylméthylcystéine, la S-benzylcystéamine, leurs sels et leurs dérivés, la tioxolone ; des agents anti-acnéiques comme le peroxyde de benzoyle ; des antibiotiques, comme l'érythromycine et ses esters, la néomycine, les tétracyclines ou les polyméthylène-4,5 isothiazolones-3 ; des agents favorisant la repousse des cheveux vendus sous la dénomination commerciale "MINOXIDIL" et ses dérivés, l'anthraline et ses dérivés, le chloro-7 méthyl-3 benzothiadiazine-1,2,4 dioxyde-1,1 vendu sous la dénomination commerciale "DIAZOXIDE", le diphényl-5,5 imidazolidinedione-2,4 vendu sous la dénomination commerciale "PHENITOIN", l'iodure d'oxapropanium ou l'acide rétinoïque et ses dérivés rétinoïdes ; des agents anti-inflammatoires stéroïdiens et non-stéroïdiens ; des caroténoïdes et, notamment, le $\beta$-carotène ; des agents anti-psoriasiques tels que l'anthraline et ses dérivés, les acides eicosatétraynoïque-5,8,11,14 et -triynoïque-5,8,11, leurs esters et leurs amides.

Les compositions selon l'invention peuvent également contenir des agents d'amélioration de la saveur, des agents conservateurs, des agents stabilisants, des agents régulateurs d'humidité, des agents régulateurs de pH, des agents émulsionnants, des filtres UV-B et UV-A, des antioxydants tels que l'$\alpha$-tocophérol, le butylhydroxyanisole ou le butylhydroxy-toluène.

Pour mieux faire comprendre l'objet de l'invention, on va en décrire maintenant plusieurs exemples de réalisation.

EXEMPLE 1

Préparation du composé de formule (I) ci-dessous où A est un groupement (IV) et où B est $CO_2R_8$ avec $R_8$ = $CH_3$ :

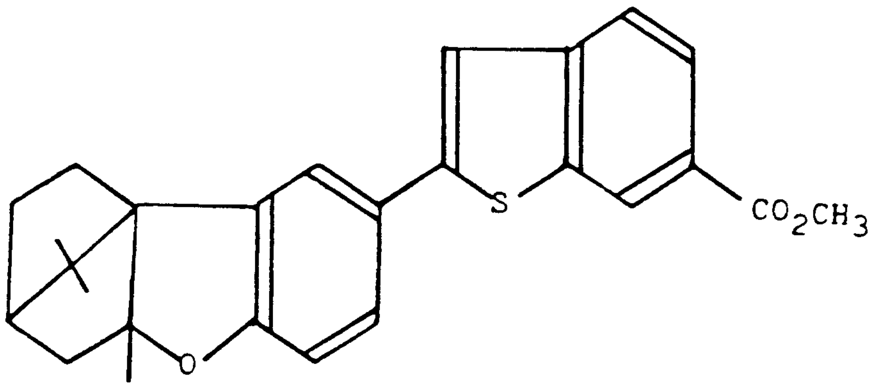

Cette préparation se fait en cinq étapes notées a) à e).

a) préparation du composé de départ : le 2,3,4,4a-tétrahydro-4a,10,10-triméthyl-1H-3,9b méthano-dibenzofuranne (TTMDBF) de formule :

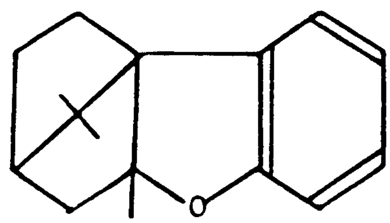

Ce produit est obtenu selon le mode opératoire décrit par J.L. FRY et W.J. WEST, J. Org. Chem. 1981, v. 46 (10) 2177-2179.

b) préparation d'un dérivé cétonique de formule ci-dessous à partir du TTMDBF

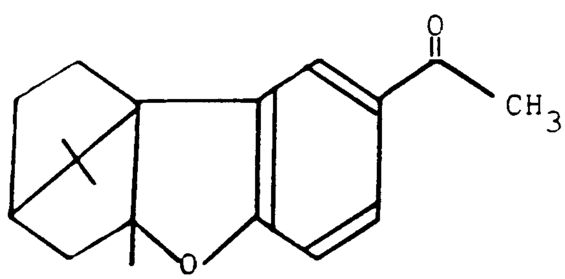

On ajoute lentement une solution de 9,2 g de TTMDBF et 3,2 cm³ de chlorure d'acétyle dans 60 cm³ de dichlorométhane à une solution préalablement refroidie à -10 °C de 6 g de chlorure d'aluminium dans 60 cm³ de dichlorométhane. On agite pendant deux heures en laissant le mélange réactionnel revenir à température ambiante. On verse sur une solution saturée de chlorure d'ammonium et extrait la phase organique à l'éther. Les phases organiques sont réunies, séchées sur sulfate de sodium et le solvant est

distillé sous pression réduite. Le résidu obtenu est recristallisé dans l'éthanol.

. Point de fusion : 139 ° C

. Spectre UV (chloroforme) : λ max = 291 nm ε = 14230

L'analyse du produit obtenu donne les résultats suivants :

| Analyse | Calculé pour $C_{18}H_{22}O_2$ | Trouvé |
|---|---|---|
| C | 79,96 | 80,01 |
| H | 8,20 | 8,20 |
| O | 11,84 | 11,68 |

c) préparation de l'acide correspondant au dérivé cétonique préparé au stade b), de formule :

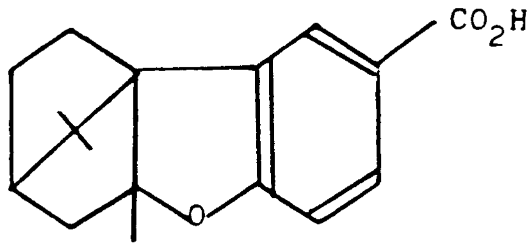

On ajoute, en maintenant la température à 0 ° C, 18 $cm^3$ de brome à une solution de 42 g de soude dans 210 $cm^3$ d'eau. Après 15 minutes, on ajoute 16,5 g de dérivé cétonique obtenu au stade b) en solution dans 100 $cm^3$ de dioxanne. On agite pendant 15 minutes à 0 ° C puis on laisse revenir à température ambiante. On chauffe pendant 2 heures à 50 ° C. Après refroidissement, on ajoute 18,1 g de métabisulfite de sodium en solution dans 140 $cm^3$ d'eau puis, goutte à goutte, 84 $cm^3$ d'acide chlorhydrique concentré. On extrait à l'éther. Après distillation du solvant sous pression réduite et recristallisation dans l'acétate d'éthyle, on obtient 12 g de produit attendu sous forme de cristaux blancs possédant les caractéristiques suivantes :

. Point de fusion : >260 ° C

. Spectre UV ($CHCl_3$) : λ max = 270 nm ε = 12000

L'analyse du produit obtenu donne les résultats suivants :

| Analyse | Calculé pour $C_{17}H_{20}O_3$ | Trouvé |
|---|---|---|
| C | 74,97 | 74,90 |
| H | 7,40 | 7,43 |
| O | 17,62 | 17,49 |

d) préparation du composé de formule :

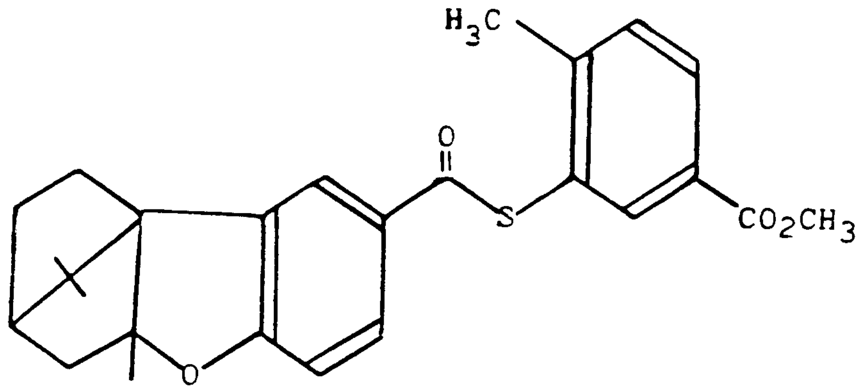

par réaction de l'acide obtenu au stade c) avec un thiol.

On chauffe pendant 3 heures à 70 °C, un mélange de 5 g de composé obtenu au stade c), 1,2 $cm^3$ de trichlorure de phosphore et 50 $cm^3$ de toluène. Le mélange réactionnel est filtré sur une terre de diatomée vendue sous la marque "CELITE" par MANVILLE PRODUCTS CORPORATION et le solvant est distillé sous pression réduite. Le résidu est redissous dans 50 $cm^3$ de tétrahydrofuranne. On ajoute 3,5 g de méthoxycarbonyl-5-méthyl-2 thiophénol et 3 $cm^3$ de triéthylamine en solution dans 20 $cm^3$ de tétrahydrofuranne.

Après une heure d'agitation, on verse le mélange réactionnel dans l'eau. On extrait à l'acétate d'éthyle. La phase organique est lavée par une solution saturée de bicarbonate de sodium puis par de la soude diluée et finalement à l'eau. Après séchage de la phase organique, distillation du solvant sous pression réduite et recristallisation dans l'éthanol, on obtient 5,8 g de produit attendu possédant les caractéristiques suivantes :

. Point de fusion : 145 °C

. Spectre UV ($CHCl_3$) : $\lambda$ max = 305 nm $\epsilon$ = 22800

L'analyse du produit obtenu donne les résultats suivants :

| Analyse | Calculé pour $C_{26}H_{28}O_4S$ | Trouvé |
|---|---|---|
| C | 71,53 | 71,41 |
| H | 6,46 | 6,32 |
| O | 14,66 | 14,57 |
| S | 7,34 | 7,25 |

e) préparation du composé de formule ci-dessous par bromuration et cyclisation du produit obtenu au stade d).

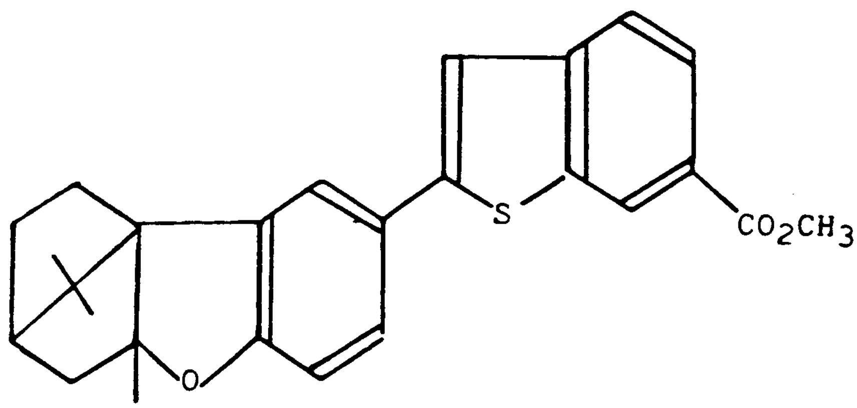

On chauffe au reflux pendant 3 heures un mélange de 2,5 g de composé obtenu au stade d) et 1,13 g de N-bromosuccinimide dans 100 $cm^3$ de tétrachlorure de carbone sous rayonnement UV. On laisse refroidir puis on filtre le succinimide formé. Le solvant est distillé sous pression réduite et le résidu dissous dans 50 $cm^3$ de toluène est filtré rapidement sur gel de silice. La phase organique est diluée par 50 $cm^3$ de toluène. On ajoute 1,5 g de triphénylphosphine et on agite pendant 4 heures à 100 ° C. Le solvant est distillé et le résidu est traité par 100 $cm^3$ d'éther isopropylique. Après filtration et évaporation du solvant, le résidu est mis en suspension dans 100 $cm^3$ de tétrahydrofuranne. On ajoute goutte à goutte 0,86 $cm^3$ de diazabicycloundécène et on agite pendant 2 heures à température ambiante. Après évaporation du solvant et recristallisation dans l'éthanol, on obtient 0,6 g de produit attendu possédant les caractéristiques suivantes :

. Point de fusion : 156 ° C

. Spectre UV($CHCl_3$) : $\lambda max$ = 342 nm $\epsilon$ = 26100

L'analyse du produit obtenu donne les résultats suivants :

| Analyse | Calculé pour $C_{26}H_{26}O_3S$ | Trouvé |
|---|---|---|
| C | 74,61 | 74,82 |
| H | 6,26 | 6,27 |
| O | 11,47 | 11,61 |
| S | 7,66 | 7,61 |

EXEMPLE 2

Préparation du composé de formule (I) ci-dessous où A est un groupement (IV), où B est $COOR_8$ avec $R_8$ = H.

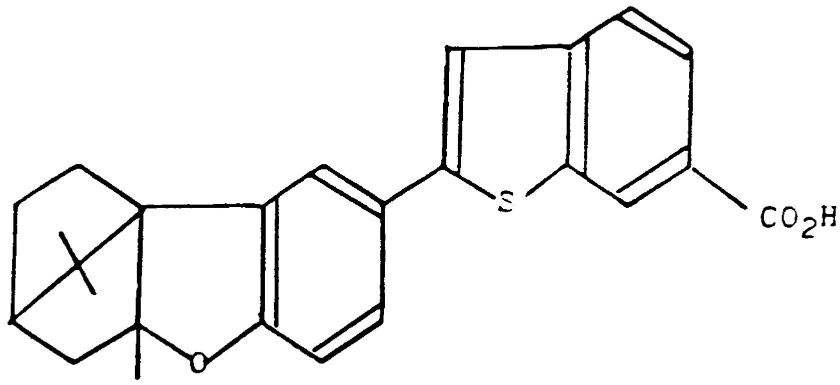

On chauffe pendant 2 heures à 70 °C un mélange de 1,9 g du composé obtenu dans l'étape e) de l'exemple 1 et 2 g de potasse dans 200 $cm^3$ d'éthanol et 50 $cm^3$ d'eau. Après distillation de l'éthanol et dilution à l'eau, on acidifie par addition d'acide chlorhydrique 1N. On filtre, lave à l'eau et sèche le précipité obtenu. Après recristallisation dans un mélange éthanol/tétrahydrofuranne, on obtient 1,45 g de produit attendu possédant les caractéristiques suivantes :

. Point de fusion : >260 °C

. Spectre UV (MeOH) : $\lambda max$ = 331 nm $\epsilon$ = 27800

L'analyse du produit obtenu donne les résultats suivants :

| Analyse | Calculé pour $C_{25}H_{24}O_3S$,0,5$H_2O$ | Trouvé |
|---|---|---|
| C | 72,55 | 72,81 |
| H | 6,05 | 6,04 |
| O | 13,54 | 13,03 |
| S | 7,74 | 7,57 |

EXEMPLE 3

Préparation du composé de formule (I) ci-dessous où A est un groupement (III), où B est $COOR_8$ avec $R_8$ = H.

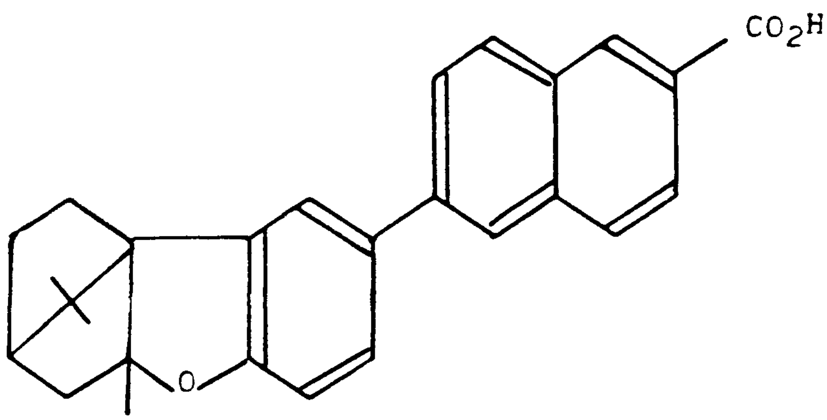

a) préparation à partir du TTMDBF, du composé bromé de formule :

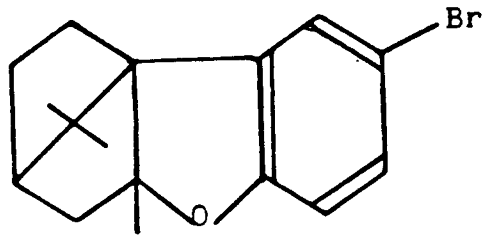

On met en suspension 22,8 g du TTMDBF obtenu au stade a) de l'exemple 1 dans 50 $cm^3$ de tétrahydrofuranne. On ajoute à 15 °C une solution de 17,8 g de N-bromosuccinimide dans 50 $cm^3$ de diméthylformamide, en 40 minutes. La température monte lentement de 15 à 30 °C. Un précipité apparaît peu à peu. Le mélange réactionnel est versé dans 500 $cm^3$ d'eau. Après extraction par 500 $cm^3$ de dichlorométhane et distillation du solvant sous pression réduite, on obtient 22 g de produit attendu sous forme d'une poudre blanche.

b) préparation du composé de formule :

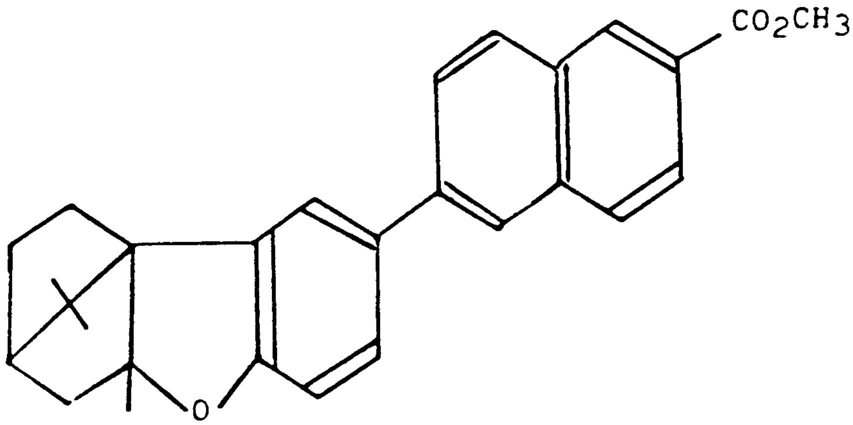

On chauffe au reflux sous azote pendant 2 heures un mélange contenant 18,4 g de composé obtenu au stade a) du présent exemple, 2,2 g de magnésium et un cristal d'iode dans 200 $cm^3$ de tétrahydrofuranne anhydre. On refroidit à 20 °C et on ajoute 13,5 g de bromure de zinc. On agite pendant 1 heure à température ambiante puis on ajoute 5,4 g de bromo-6-naphtalène carboxylate de méthyle et 0,1 g de catalyseur $NiCl_2$-1,2- (diphénylphosphino)éthane (DPPE). On agite pendant 1 heure à température ambiante, puis on verse le mélange réactionnel dans une solution saturée de chlorure d'ammonium. Après extraction à l'acétate d'éthyle et distillation du solvant, le résidu est recristallisé dans un mélange acétonitrile/heptane. On obtient ainsi 7g de produit attendu sous forme de cristaux blancs :

. Point de fusion : 210 °C
. Spectre UV ($CH_2Cl_2$) : $\lambda max = 330$ nm $\epsilon = 23700$

L'analyse du produit obtenu donne les résultats suivants :

| Analyse | Calculé pour $C_{28}H_{28}O_3$ | Trouvé |
|---|---|---|
| C | 81,52 | 81,54 |
| H | 6,84 | 6,83 |
| O | 11,64 | 11,49 |

c) préparation du composé de formule :

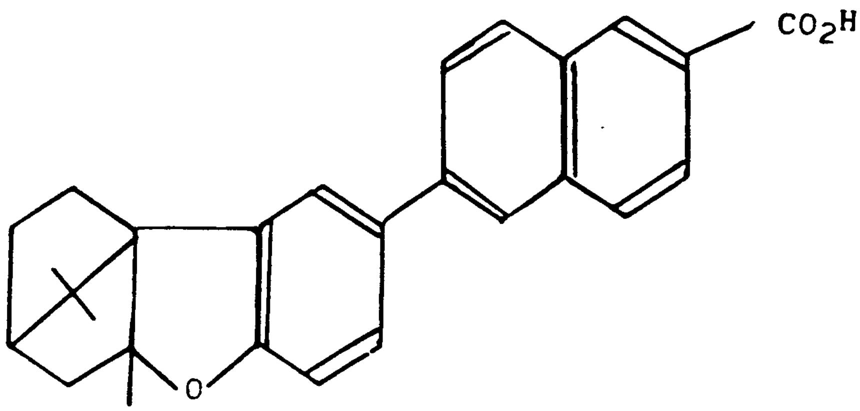

On chauffe pendant 2 heures à 70 °C, un mélange contenant 6,5 g de composé obtenu au stade b) du présent exemple et 2 g de potasse dans 200 $cm^3$ d'éthanol et 50 $cm^3$ d'eau. Après distillation de l'éthanol et dilution à l'eau, on acidifie par addition d'acide chlorhydrique 1N. On filtre, lave à l'eau et sèche le précipité obtenu. Après recristallisation dans un mélange d'éthanol et de tétrahydrofuranne, on obtient 5 g de produit attendu.

Le produit obtenu possède les caractéristiques suivantes :

. Point de fusion : 250 °C

. Spectre UV ($CH_2Cl_2$) : λmax = 330 nm ε = 23500

L'analyse du produit obtenu donne les résultats suivants :

| Analyse | Calculé pour $C_{27}H_{26}O_3$ | Trouvé |
|---|---|---|
| C | 81,38 | 81,60 |
| H | 6,57 | 6,60 |
| O | 12,05 | 12,29 |

EXEMPLE 4

## Préparation du composé de formule (I) ci-dessous où A est un groupement (III) et où B a pour formule $CO-R_5$, $R_5$ étant 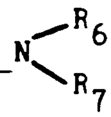 avec $R_6 = H$ et $R_7 = C_2H_5$

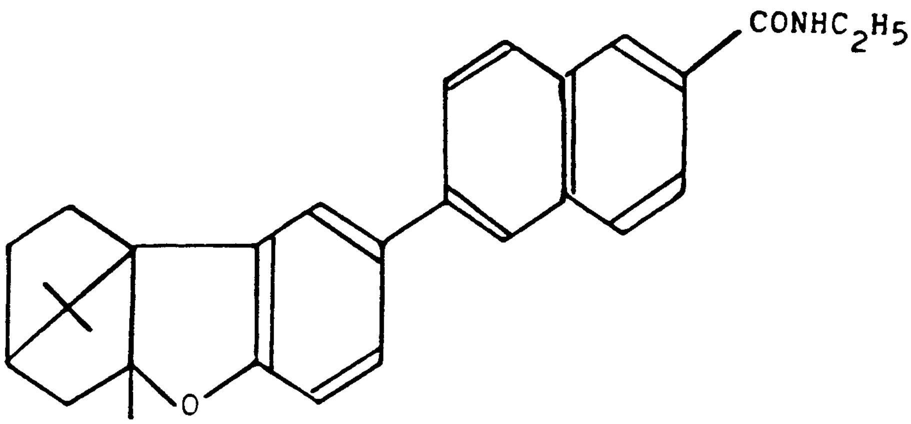

On agite pendant 30 mn une suspension de 1 g (2,5 mmoles) d'acide obtenu à l'exemple 3 ci-dessus et 0,45 g de N,N'-carbonyldiimidazole dans 40 $cm^3$ de dichlorométhane. Le solvant est distillé sous pression réduite. On refroidit à 0 °C puis on ajoute 40 $cm^3$ de tétrahydrofuranne et 1 $cm^3$ d'éthylamine anhydre. On agite pendant 1 heure puis on verse le mélange réactionnel dans l'eau. On extrait à l'acétate d'éthyle. La phase organique est lavée à l'eau puis séchée sur sulfate de sodium. Après distillation du solvant sous pression réduite, le résidu est recristallisé dans un mélange éthanol-eau.

Le produit obtenu possède les caractéristiques suivantes :

. Point de fusion : >260 °C

. Spectre UV ($CH_2Cl_2$) : λ max : 330 nm ε : 24000

L'analyse du produit obtenu donne les résultats suivants :

| Analyse | Calculé pour ($C_{29}H_{31}NO_2, \frac{1}{3}\ H_2O$) | Trouvé |
|---|---|---|
| C | 80,74 | 80,64 |
| H | 7,35 | 7,36 |
| N | 3,25 | 3,27 |
| O | 8,66 | 8,38 |

EXEMPLE 5

Préparation du composé de formule (I) ci-dessous, où A est un groupement (V) avec $R_3$ = $CH_3$ et D = S-$CH_3$

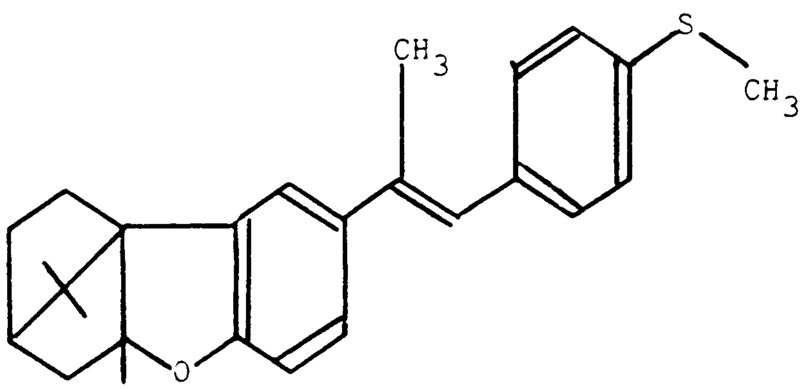

On met en suspension 5,4 g de produit obtenu au stade b) dans l'exemple 1 dans 30 $cm^3$ de méthanol et 2 $cm^3$ d'eau. On ajoute 0,6 g de borohydrure de sodium et on agite à température ambiante pendant 16 heures. Le mélange réactionnel est versé sur de la glace. Le précipité et la phase aqueuse sont extraits trois fois à l'aide de 60 $cm^3$ d'éther. La phase organique est séchée sur du sulfate de sodium et le solvant est distillé sous pression réduite. On obtient 5,7 g de produit que l'on redissout dans 50 $cm^3$ d'éther de pétrole. La solution est refroidie vers -10 °C et on ajoute en 30 minutes environ 1,83 g de tribromure de phosphore en maintenant la température au-dessous de -10 °C. Après l'addition du tribromure de phosphore, on agite pendant 12 heures à température ambiante. Le mélange réactionnel est versé sur de la glace. On extrait trois fois à l'aide de 60 $cm^3$ d'éther. La phase organique est lavée par une solution aqueuse de bicarbonate de sodium puis par une solution saturée de chlorure de sodium. Après séchage sur du sulfate de sodium puis évaporation du solvant, on récupère 5,5 g de cristaux jaunes, qui sont redissous dans 50 $cm^3$ de toluène. On ajoute 4,1 g de triphénylphosphine, on agite pendant 12 heures à température ambiante, puis pendant 6 heures à 55 °C. On laisse refroidir puis on filtre le précipité ; on le lave à l'hexane et on le sèche. On obtient 6,5 g de produit jaune pâle, qui est redissous dans 100 $cm^3$ de méthanol. On ajoute 2 g de carbonate de potassium et 1,1 g de méthylthio-4-benzaldéhyde. On chauffe au reflux pendant 4 heures, puis on verse le mélange réactionnel dans l'eau. Après extraction à l'acétate d'éthyle et distillation du solvant sous pression réduite, on obtient un résidu huileux qui, après recristallisation dans l'éthanol à 95%, possède les caractéristiques suivantes :

. Point de fusion : 130 °C
. Spectre UV ($CH_2Cl_2$) : λmax = 309 nm ε = 30200

L'analyse du produit obtenu donne les résultats suivants :

| Analyse | Calculé pour $C_{26}H_{30}OS$ | Trouvé |
|---|---|---|
| C | 79,95 | 79,89 |
| H | 7,74 | 7,68 |
| O | 4,10 | 4,26 |
| S | 8,21 | 8,11 |

EXEMPLE 6

Préparation du composé de formule (I) ci-dessous, où A est un groupement (II), où $R_1$ et $R_2$ forment un groupe oxo = O et B = $COR_5$ $R_5$ étant -O-$R_8$ avec $R_8$ = $CH_3$

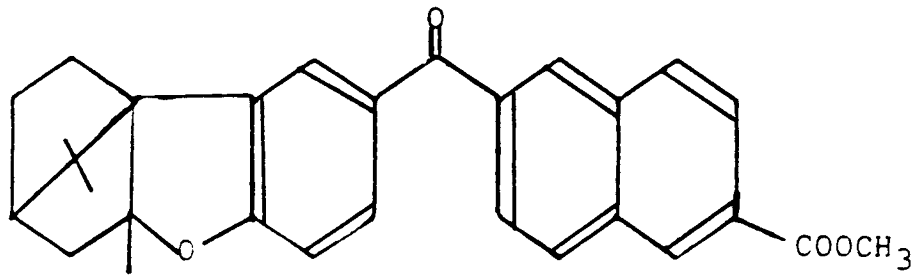

A une suspension de 2,39 g (10 mmoles) de TTMDBF préparé au stade a) de l'exemple 1 et de 2,49 g (10 mmoles) de chlorure de l'acide méthoxycarbonyl-6 naphtalène carboxylique-2 dans 50 $cm^3$ de dichloro-1,2 éthane anhydre, on ajoute par portions en 45 minutes, 2 g de chlorure d'aluminium anhydre. Le mélange est agité pendant 4 heures à température ambiante puis versé dans 20 $cm^3$ d'eau glacée. La phase organique est décantée. La phase aqueuse est à nouveau extraite par 50 $cm^3$ de dichloroéthane. Les phases de dichloroéthane sont rassemblées, lavées à l'eau, séchées sur sulfate de sodium et concentrées sous pression réduite. L'huile brute obtenue est purifiée par chromatographie sur gel de silice 60 avec élution au dichlorométhane puis à l'acétate d'éthyle. Le solide blanc isolé est recristallisé dans le minimum d'hexane. On obtient ainsi, après séchage, 2,9 g de [(TTMDBF) carbonyl]-6 naphtalène carboxylate de méthyle-2 ayant les caractéristiques suivantes :

. Point de fusion : 146-148 °C

. RMN $^1H$ 80MHZ conforme à la structure attendue.

L'analyse du produit obtenu donne les résultats suivants :

| Analyse | Calculé pour $C_{29}H_{28}O_4$ | Trouvé |
|---|---|---|
| C | 79,06 | 78,96 |
| H | 6,41 | 6,43 |
| O | 14,53 | 14,37 |

EXEMPLE 7

Préparation du composé de formule (I) ci-dessous, où A est un groupement (II), $R_1$ et $R_2$ formant ensemble un groupe oxo, B étant $-\underset{\underset{O}{\parallel}}{C}-R_5$ avec $R_5 = OR_8$ et $R_8 = H$

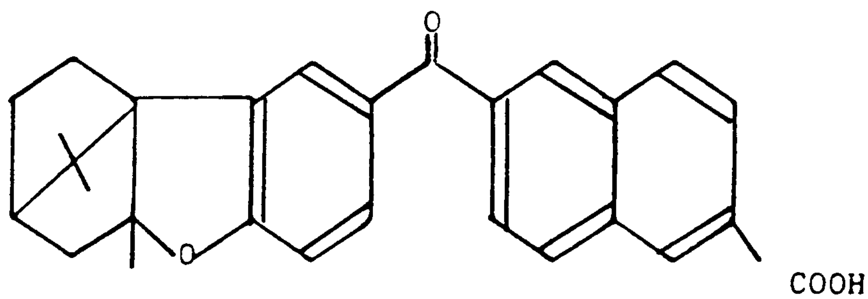

Une suspension de 1,9 g (4,3 mmoles) de [(TTMDBF) carbonyl]-6 naphtalène carboxylate de méthyle-2, obtenu selon l'exemple 6, est agitée pendant 2 heures dans un mélange de 25 $cm^3$ d'alcool et de 25 $cm^3$ de potasse aqueuse 6N chauffé au reflux. Après addition de 100 $cm^3$ d'eau, l'alcool est éliminé par évaporation sous vide. La phase aqueuse est diluée à environ 300 $cm^3$, puis acidifiée par 20 $cm^3$ d'acide chlorhydrique 12N. Le précipité obtenu est essoré, lavé abondamment à l'eau et séché sur potasse à 80 °C-100 °C. Après une première recristallisation dans le méthanol, en présence de charbon animal, puis une seconde recristallisation également dans le méthanol, on obtient 1,25 g de cristaux blancs d'acide [-(TTMDBF) carbonyl]-6 naphtalène carboxylique-2 ayant les caractéristiques suivantes :

. Point de fusion : 271 °C

. RMN $^1H$ 250 MHZ conforme à la structure.

L'analyse du produit obtenu donne les résultats suivants :

| Analyse | Calculé pour $C_{28}H_{26}O_4$ | Trouvé |
|---|---|---|
| C | 78,85 | 78,76 |
| H | 6,14 | 6,16 |
| O | 15,00 | 15,15 |

EXEMPLE 8

Préparation du composé de formule (I) ci-dessous, où A est un groupement (II), $R_1$ et $R_2$ formant ensemble un groupe oxo, B étant $COR_5$,

$R_5$ étant un radical 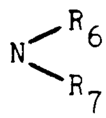 avec $R_6$ = H et $R_7$ = $CH_2CH_3$

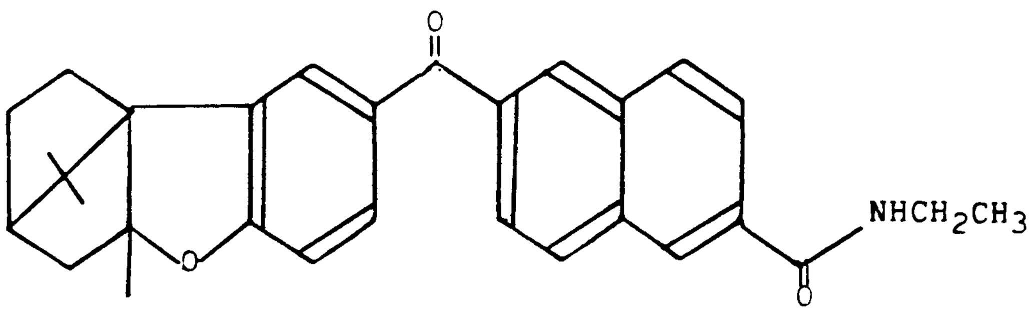

Une suspension de 260 mg (0,6 mmole) d'acide [(TTMDBF) carbonyl]-6 naphtalène carboxylique-2, obtenu à l'exemple 7, et de 120 mg (0,72 mmole) de N,N'-carbonyldiimidazole dans 5 $cm^3$ de dichloro-1,2 éthane anhydre est agitée 1 heure à température ambiante. La solution obtenue est refroidie à 0 °C et on ajoute 0,05 $cm^3$ (0,75 mmole) d'éthylamine anhydre. L'agitation est maintenue pendant 2 heures en laissant revenir à la température ambiante. Le milieu réactionnel est alors dilué par 20 $cm^3$ de dichloroéthane, lavé à l'acide chlorhydrique 0,1 N puis à l'eau. La phase organique est séchée sur sulfate de sodium et évaporée à sec. L'amide brut est recristallisé dans l'hexane contenant une trace d'acétone. Après séchage, on obtient 210 mg de cristaux blancs de N-éthyl [(TTMDBF) carbonyl]-6 naphtalène carboxamide-2 ayant les caractéristiques suivantes :

. Point de fusion : 123-125 °C

. RMN $^1H$ 250 MHZ conforme à la structure attendue.

L'analyse du produit obtenu donne les résultats suivants :

| Analyse | Calculé pour $C_{30}H_{31}NO_3$ | Trouvé |
|---|---|---|
| C | 79,44 | 79,39 |
| H | 6,89 | 6,98 |
| N | 3,09 | 3,10 |
| O | 10,58 | 10,61 |

EXEMPLE 9

On prépare un gel en réalisant la formulation suivante :

| | |
|---|---|
| - Composé de l'exemple 3c | 0,05 g |
| - Erythromycine base | 4,000 g |
| - Butylhydroxytoluène | 0,050 g |
| - Hydroxypropylcellulose vendue par la Société Hercules sous le nom de "KLUCEL HF" | 2,000 g |
| - Ethanol (à 95°) .... q.s.p. | 100,000 g |

Ce gel est appliqué sur une peau à dermatose ou une peau acnéique 1 à 3 fois par jour et on constate une amélioration significative dans un délai compris entre 6 et 12 semaines selon la gravité du cas traité.

EXEMPLE 10

On prépare la formulation suivante destinée à être conditionnée dans une gélule :

| | |
|---|---|
| - Composé de l'exemple 3c | 0,06 g |
| - Amidon de maïs | 0,060 g |
| - Lactose ........... q.s.p. | 0,3000 g |

Les gélules utilisées sont constituées de gélatine, d'oxyde de titane et d'un conservateur.

On administre à un individu adulte 1 à 3 gélules par jour dans le traitement du psoriasis et on constate une amélioration significative au bout de 30 jours environ.

EXEMPLE 11

On prépare une lotion antiseborrhéique en procédant au mélange des ingrédients suivants :

| | |
|---|---|
| - Composé de l'exemple 1 | 0,03 g |
| - Propylène glycol | 5,00 g |
| - Butylhydroxytoluène | 0,10 g |
| - Ethanol (à 95°)......q.s.p. | 100,00 g |

Cette lotion est appliquée deux fois par jour sur un cuir chevelu séborrhéique et on constate une amélioration significative dans un délai compris entre 2 et 6 semaines.

EXEMPLE 12

On prépare une composition cosmétique antisolaire en procédant au mélange des ingrédients suivants :

| | |
|---|---|
| - Composé de l'exemple 6 | 1 g |
| - Benzylidène camphre | 4 g |
| - Triglycérides d'acides gras | 31 g |
| - Monostéarate de glycérol | 6 g |
| - Acide stéarique | 2 g |
| - Alcool cétylique | 1,2 g |
| - Lanoline | 4,0 g |
| - Conservateurs | 0,3 g |
| - Propanediol | 2,0 g |
| - Triéthanolamine | 0,5 g |
| - Parfum | 0,4 g |
| - Eau déminéralisée .. q.s.p. | 100,0 g |

EXEMPLE 13

On prépare un gel pour application topique en procédant au mélange des ingrédients suivants :

| | |
|---|---|
| - Composé de l'exemple 4 | 0,05 g |
| - Ethanol | 43,00 g |
| - α-tocophérol | 0,05 g |
| - Polymère carboxyvinylique vendu sous la dénomination "Carbopol 941" par la Société "Goodrich"... | 0,50 g |
| - Triéthanolamine en solution aqueuse à 20 % en poids | 3,80 g |
| - Eau | 9,30 g |
| - Propylène glycol.....q.s.p. | 100,00 g |

Dans cet exemple, le composé de l'exemple 4 peut être remplacé, dans les mêmes proportions, par celui de l'exemple 6.

Ce gel est appliqué sur une peau à dermatose ou une peau acnéique 1 à 3 fois par jour. On constate une amélioration significative dans un délai compris entre 6 et 12 semaines selon la gravité du cas traité.

EXEMPLE 14

On prépare une crème anti-acnéique en procédant au mélange des ingrédients suivants :

| | |
|---|---|
| - Mélange de stéarates de glycérol et de polyéthylène glycol (75 moles) vendu sous le nom de "Gelot 64" par la Société "GATTEFOSSE" | 15 g |
| - Huile de noyau polyoxyéthylénée à 6 moles d'oxyde d'éthylène vendue sous le nom de "Labrafil M 2130 CS" par la Société "GATTEFOSSE" | 8 g |
| - Perhydrosqualène | 10 g |
| - Colorant .......... q.s. | |
| - Conservateurs ..... q.s. | |
| - Parfums .......... q.s. | |
| - Tioxolone | 0,4 g |
| - Polyéthylèneglycol (masse moléculaire = 400) | 8 g |
| - Eau purifiée | 58,5 g |
| - Sel disodique de l'acide éthylène-diamine tétracétique | 0,05 g |
| - Composé de l'exemple 7 | 0,05 g |

Cette crème est appliquée sur une peau à dermatose ou une peau acnéique 1 à 3 fois par jour. On constate une amélioration significative dans un détail compris entre 6 et 12 semaines selon la gravité du cas traité.

EXEMPLE 15

On prépare une lotion capillaire anti-chute et pour la repousse des cheveux en procédant au mélange des ingrédients suivants :

| | |
|---|---|
| - Propylèneglycol | 20 g |
| - Ethanol | 34,92 g |
| - Polyéthylèneglycol (masse moléculaire = 400) | 40 g |
| - Eau | 4 g |
| - Butylhydroxyanisole | 0,01 g |
| - Butylhydroxytoluène | 0,02 g |
| - Composé de l'exemple 3c | 0,05 g |
| - Composé vendu sous la dénomination commerciale "Minoxidil" | 1 g |

On applique cette lotion 2 fois par jour sur un cuir chevelu ayant subi une chute de cheveux importante. Après 3 mois de traitement, on observe une amélioration significative.

Dans cet exemple de formulation, le composé de l'exemple 3c peut être remplacé par celui de l'exemple 8.

EXEMPLE 16

On prépare un comprimé non soluble de 0,5 g en procédant au mélange des substances suivantes :

| | |
|---|---|
| - Composé de l'exemple 7 | 0,025 g |
| - Lactose | 0,082 g |
| - Acide stéarique | 0,003 g |
| - Talc purifié | 0,015 g |
| - Edulcorant...........q.s. | |
| - Colorant............q.s. | |
| - Amidon de riz........q.s.p. | 0,500 g |

On administre à un individu adulte atteint de psoriasis 1 à 3 comprimés par jour par voie orale et on constate une amélioration significative au bout de 30 jours environ.

EXEMPLE 17

On prépare une solution à 0,20 % en poids d'actif en réalisant la formulation suivante :

| | |
|---|---|
| - Composé de l'exemple 8 | 0,2 g |
| - Polyéthylèneglycol (masse moléculaire = 400) | 80,0 g |
| - Ethanol (à 95°).......q.s.p. | 100,0 g |

Cette solution est appliquée sur une peau acnéique 1 à 3 fois par jour et on constate une amélioration significative dans un délai compris entre 6 et 12 semaines selon la gravité du cas traité.

EXEMPLE 18

On prépare une crème anti-séborrhéique en réalisant la formulation suivante :

| | |
|---|---|
| - Stéarate de polyoxyéthylène (40 moles d'oxyde d'éthylène) vendu sous le nom de "Myrj 52" par la Société "ATLAS" | 4 g |
| - Monolaurate de sorbitan, polyoxyéthyléné à 20 moles d'oxyde d'éthylène, vendu sous le nom de "Tween 20" par la Société "ATLAS" | 1,8 g |
| - Mélange de mono et distéarate de glycérol vendu sous la dénomination de "GELEOL" par la Société "GATTEFOSSE" | 4,2 g |
| - Propylèneglycol | 10,0 g |
| - Butylhydroxyanisole | 0,01 g |
| - Butylhydroxytoluène | 0,02 g |
| - Alcool céto-stéarylique | 6,2 g |
| - Conservateurs ..... q.s. | |
| - Perhydrosqualène | 18,0 g |
| - Mélange de triglycérides caprylique - caprique vendu sous la dénomination de "Miglyol 812" par la Société "DYNAMIT NOBEL" | 4 g |
| - S-carboxyméthyl cystéine | 3 g |
| - Triéthanolamine (99 % en poids) | 2,5 g |
| - Composé de l'exemple 3c | 0,02 g |
| - Eau.....................q.s.p. | 100,00 g |

Cette crème est appliquée 2 fois par jour sur une peau à tendance grasse et on constate une amélioration significative dans un délai compris entre 2 et 6 semaines.

EXEMPLE 19

On prépare une crème anti-séborrhéique en réalisant la formulation suivante :

| | |
|---|---|
| - Stéarate de polyoxyéthylène (40 moles d'oxyde d'éthylène) vendu sous le nom de "Myrj 52" par la Société "ATLAS" | 4 g |
| - Monolaurate de sorbitan polyoxyéthyléné à 20 moles d'oxyde d'éthylène vendu sous le nom de "Tween 20" par la Société "ATLAS" | 1,8 g |
| - Mélange de mono et distéarate de glycérol vendu sous la dénomination de "GELEOL" par la Société "GATTEFOSSE" | 4,2 g |
| - Propylèneglycol | 10,0 g |
| - Butylhydroxyanisole | 0,01 g |
| - Butylhydroxytolùene | 0,02 g |
| - Alcool céto-stéarylique | 6,2 g |
| - Conservateurs ..... q.s. | |
| - Perhydrosqualène | 18 g |
| - Mélange de triglycérides caprylique - caprique vendu sous la dénomination de "Miglyol 812" par la Société "DYNAMIT NOBEL" | 4 g |
| - Amino-5-carboxy-5 thia-3 pentanoate de benzylthio-2 éthylammonium | 3 g |
| - Composé de l'exemple 3b | 0,02 g |
| - Eau................q.s.p. | 100 g |

Cette crème est appliquée 2 fois par jour sur une peau à tendance grasse et on constate une amélioration significative dans un délai compris entre 2 et 6 semaines.

EXEMPLE 20

On prépare une lotion pour la repousse des cheveux en procédant au mélange des ingrédients suivants :

| | |
|---|---|
| - Propylèneglycol | 13,96 g |
| - Polyéthylèneglycol (masse moléculaire = 300) | 40 g |
| - Polyéthylèneglycol (masse moléculaire = 1500) | 32 g |
| - Isopropanol | 12 g |
| - Butylhydroxyanisole | 0,01 g |
| - Butylhydroxytoluène | 0,02 g |
| - Composé de l'exemple 5 | 0,05 g |
| - Composé vendu sous la dénomination commerciale "Minoxidil" | 2 g |

On applique la lotion sur le cuir chevelu 2 fois par jour et, après 3 mois de traitement, on constate une amélioration significative.

EXEMPLE 21

On réalise un kit anti-acné comprenant deux parties :

a) on prépare un gel en réalisant la formulation suivante :

| | |
|---|---|
| - Alcool éthylique | 48,4 g |
| - Propylèneglycol | 50 g |
| - Polymère carboxyvinylique vendu sous la dénomination commerciale "CARBOPOL 940" par la société "GOODRICH" | 1 g |
| - Diisopropanolamine (99 % en poids) | 0,3 g |
| - Butylhydroxyanisole | 0,05 g |
| - Butylhydroxytoluène | 0,05 g |
| - $\alpha$ tocophérol | 0,1 g |
| - Composé de l'exemple 5 | 0,1 g |

b) on prépare un gel en réalisant la formulation suivante :

| | |
|---|---|
| - Alcool éthylique | 5 g |
| - Propylèneglycol | 5 g |
| - Sel disodique de l'acide éthylène-diamine tétracétique | 0,05 g |
| - Polymère carboxyvinylique vendu sous la dénomination commerciale "Carbopol 940" par la Société "GOODRICH" | 1 g |
| - Triéthanolamine (99 % en poids) | 1 g |
| - Lauryl sulfate de sodium | 0,1 g |
| - Eau purifiée | 75,05 g |
| - Peroxyde de benzoyle hydraté à 25 % en poids | 12,8 g |

Le mélange des deux gels se fait extemporanément, poids pour poids, au moment de l'emploi. Le mélange obtenu est appliqué sur une peau à dermatose ou une peau acnéique 1 à 3 fois par jour et on constate une amélioration significative dans un délai compris entre 6 et 12 semaines selon la gravité du cas traité.

## Revendications

**1.** Composé benzofurannique , caractérisé par le fait qu'il a pour formule (I) :

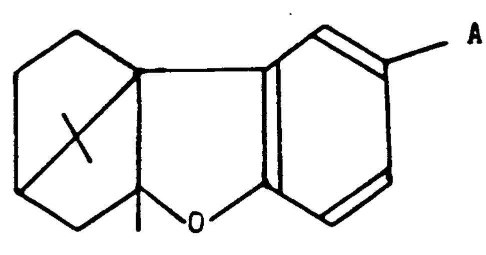

(I)

formule dans laquelle A représente :

- soit un groupement (II):

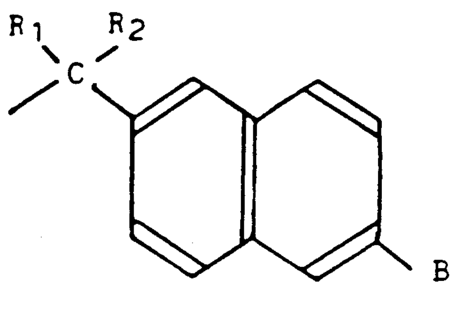

(II)

- soit un groupement (III):

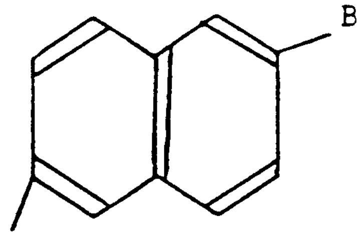

(III)

- soit un groupement (IV):

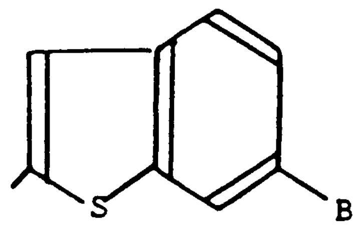

(IV)

- soit un groupement (V):

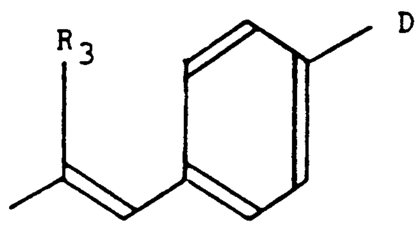

(V)

dans le groupement (II), $R_1$ représentant un atome d'hydrogène, un radical OH, un radical alcoxy en $C_1$-$C_4$, un radical acyloxy en $C_1$-$C_4$ ou un radical $NH_2$ ; $R_2$ représentant un atome d'hydrogène, ou un radical alcoxy en $C_1$-$C_4$ ; ou bien $R_1$ et $R_2$, pris ensemble, forment un radical oxo, méthano ou hydroxyimino ;

dans les groupements (II) à (IV), B représentant :

- un radical CN ;
- un radical oxazolinyle ;
- un radical correspondant à la formule :

  $-CH_2OR_4$

  formule dans laquelle $R_4$ représente un atome d'hydrogène, un radical alkyle en $C_1$-$C_6$, un radical mono- ou polyhydroxyalkyle en $C_2$-$C_6$, un radical cyclopentyle, un radical cyclohexyle ou un radical tétrahydropyrannyle .
- un radical correspondant à la formule :

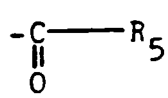

  dans laquelle $R_5$ représente :
  . un atome d'hydrogène ;
  . un radical alkyle en $C_1$-$C_6$ ;

. un radical

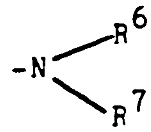

où $R^6$ et $R^7$ représentent un atome d'hydrogène, un radical alkyle en $C_1$-$C_6$, un radical alcényle en $C_3$-$C_6$, un radical cyclopentyle ou cyclohexyle ou encore un radical aralkyle ou aryle éventuellement substitués, $R^6$ et $R^7$ pouvant former un cycle avec l'atome d'azote auquel ils se rattachent, le radical

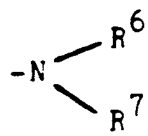

pouvant, en outre, être le reste d'un aminoacide ou d'un amino-sucre ;

. un radical -O-$R_8$, où $R_8$ représente un atome d'hydrogène, un radical alkyle en $C_1$-$C_{20}$ , un reste mono- ou polyhydroxyalkyle en $C_2$-$C_6$, le groupement -O-$R_8$ pouvant également être dérivé d'un sucre ;

- un radical D représentant soit un reste -SH, soit un reste -$S(O)_nR_9$ dans lequel n est compris entre 0 et 2, bornes incluses, $R_9$ représentant :
  . un radical alkyle en $C_1$-$C_6$ substitué ou non par un ou plusieurs groupes alcoxycarbonyle en $C_2$-$C_6$, un ou plusieurs groupements carboxyles, un ou plusieurs groupes amino ou dialkylamino ou pouvant être un reste d'un aminoacide ;
  . un radical alcényle en $C_3$-$C_6$ ;
  . un radical mono- ou polyhydroxyalcoyle en $C_2$-$C_6$ ;
  . et lorsque n est égal à 1 ou 2, un reste hydroxy, un reste alcoxy en $C_1$-$C_6$ ou un reste

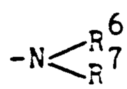

dans
lequel $R^6$ et $R^7$ peuvent avoir les significations définies ci-dessus ;

dans le groupement (V), D ayant la signification donnée ci-dessus et $R_3$ représentant un reste alkyle en $C_1$-$C_6$;
ainsi que leurs sels et isomères.

**2.** Composé selon la revendication 1, ayant une formule (I) dans laquelle A est un groupement (II), dans lequel B est soit $COOR_8$, avec $R_8$ = H ou un radical alkyle en $C_1$-$C_{20}$, soit un radical $COR_5$ avec $R_5$ =

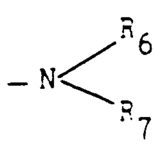 ,

$R_6$ étant H et $R_7$ étant un radical alkyle en $C_1$-$C_6$.

**3.** Composé selon la revendication 1, ayant une formule (I) dans laquelle A est un groupement (III) dans lequel B est soit $COR_5$ avec $R_5$ =

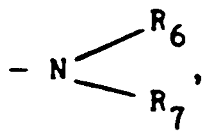

$R_6$ étant un hydrogène et $R_7$ étant un radical alkyle en $C_1$-$C_6$, soit $COOR_8$ avec $R_8$ = H ou un radical alkyle en $C_1$-$C_{20}$.

4. Composé selon la revendication 1, ayant une formule (I) dans laquelle A est un groupement (IV), où B représente $COOR_8$, $R_8$ étant H ou un radical alkyle en $C_1$-$C_{20}$.

5. Composé selon la revendication 1, ayant une formule (I) dans laquelle A est un groupement (V) et D est un reste $-SCH_3$.

6. Procédé de préparation d'un composé ayant une formule (I) selon la revendication 1, dans laquelle A est le groupement (II), $R_1$ et $R_2$ formant ensemble un radical oxo, caractérisé par le fait qu'en premier lieu, le produit (1) constitué par un acide alcoxycarbonyl-6-naphtalène carboxylique-2 est transformé en chlorure (2) correspondant par action de chlorure de thionyle ; et, en second lieu, on fait réagir le chlorure d'acide obtenu (2) avec le 2,3,4,4a-tétrahydro-4a, 10,10-triméthyl-1H-3,9b méthano-dibenzofuranne (3) soit directement par réaction de Friedel-Crafts, soit par réaction sur un dérivé magnésien halogéné (4) préalablement préparé, l'ester benzofurannique (5) obtenu pouvant être ensuite saponifié pour donner l'acide (6) correspondant.

7. Procédé selon la revendication 6, caractérisé par le fait que l'on utilise l'ester (5) ou l'acide (6) benzofurannique obtenu comme produit de départ pour la fabrication d'un autre composé ayant une formule (I), dans laquelle A est le groupement (II).

8. Procédé de préparation d'un composé ayant une formule (I) selon la revendication 1, dans laquelle A est le groupement (III), caractérisé par le fait qu'on fait une réaction de couplage entre un dérivé benzofurannique halogéné de formule :

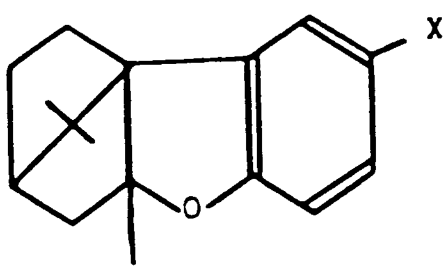

(10)

et un dérivé halogéné de naphtalène de formule :

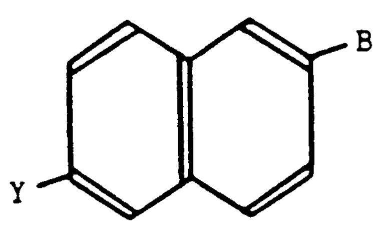

(11)

où B a la même signification que dans la revendication 1 et X et Y représentent un atome d'halogène, le dérivé halogéné (10) étant transformé en magnésien, lithien ou zincique, puis couplé avec le dérivé halogéné du naphtalène (11) en utilisant comme catalyseur un métal de transition ou l'un de ses complexes.

9. Procédé de préparation d'un composé ayant une formule (I) selon la revendication 1, dans laquelle A est un groupement (IV), caractérisé par le fait qu'on fait réagir un dérivé benzofurannique d'acide carboxylique (12) de formule :

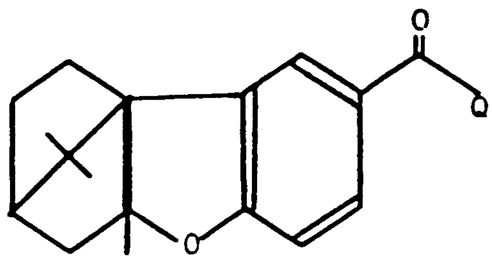

(12)

où Q = OH ou Cl
avec un thiol (13) de formule :

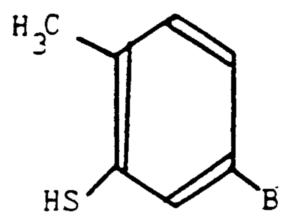

(13)

pour obtenir un produit intermédiaire (14) de formule :

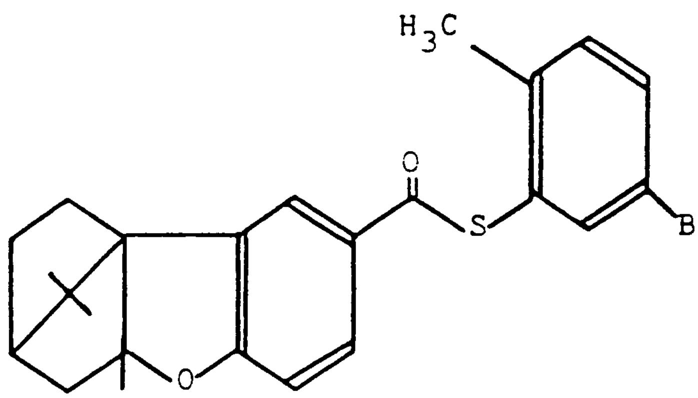

(14)

où B a la signification donnée dans la revendication 1, que le produit (14) est soumis ensuite à une bromation pour obtenir un dérivé bromométhylé de formule :

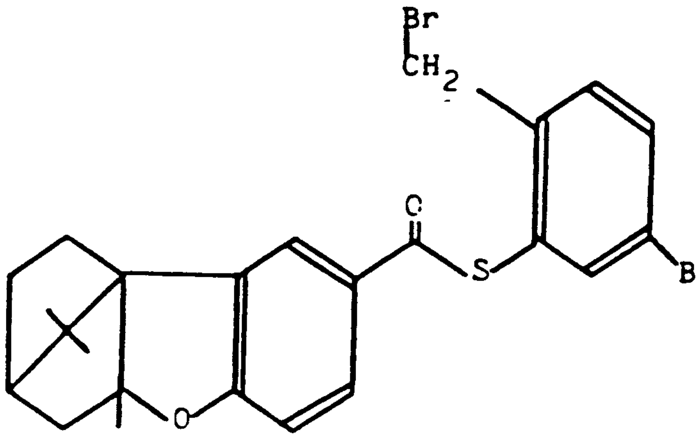

(15)

puis à une réaction avec une triaryl-phosphine et à une cyclisation pour obtenir le produit de formule :

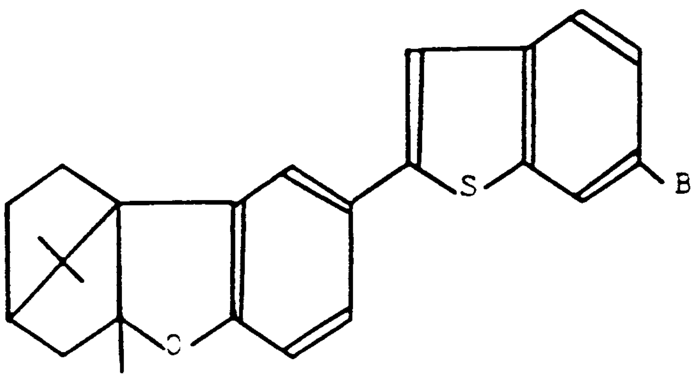

(16)

**10.** Procédé de préparation d'un composé ayant une formule (I) selon la revendication 1, dans laquelle A est un groupement (V), caractérisé par le fait qu'on fait réagir un composé benzofurannique de formule :

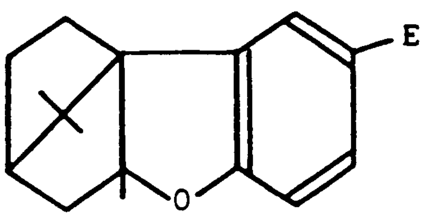

(17)

avec un composé de formule :

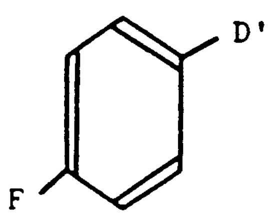

(18)

dans lesquelles D' représente un reste $-SO_3^{\ominus}M^{\oplus}$, $-SO_2^{\ominus}M^{\oplus}$, alkylthio, alkylsulfinyle ou alkylsulfonyle, dans lesquels le radical alkyle est en $C_1$-$C_6$, E représente un reste

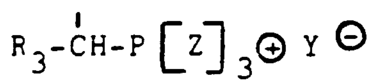

lorsque F représente un reste formyle, ou bien E représente un reste

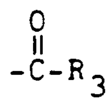

lorsque F représente un reste

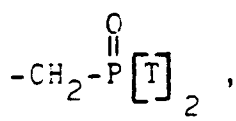

Z représentant un reste aryle, T représentant un reste alkoxy en $C_1$-$C_6$, $Y^{\ominus}$ étant un anion d'un acide minéral ou organique et $M^{\oplus}$ étant un cation d'un métal alcalin ou alcalino-terreux, $R_3$ ayant la signification définie ci-dessus.

**11.** Procédé de préparation d'un composé ayant une formule (I) selon la revendication 1, dans laquelle A est un groupement (V), caractérisé par le fait qu'on fait réagir une base forte sur le composé de formule :

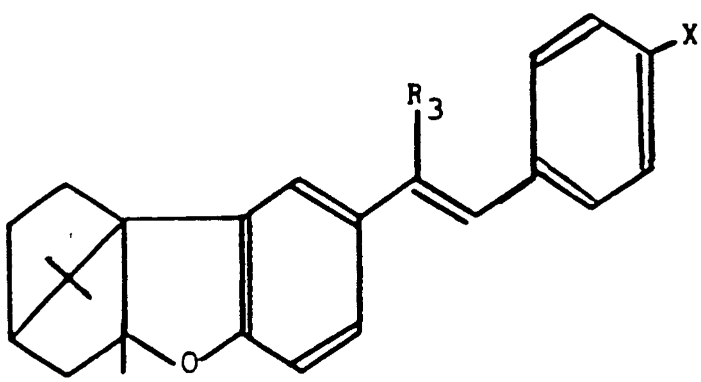

(19)

$R_3$ ayant, dans le composé (19), la signification définie ci-dessus et X représentant un atome d'halogène puis on soumet le produit obtenu à l'action de $SO_2$.

**12.** Procédé de préparation d'un composé ayant une formule (I) selon la revendication 1, dans laquelle A est un groupement (V), caractérisé par le fait qu'on fait réagir un composé de formule :

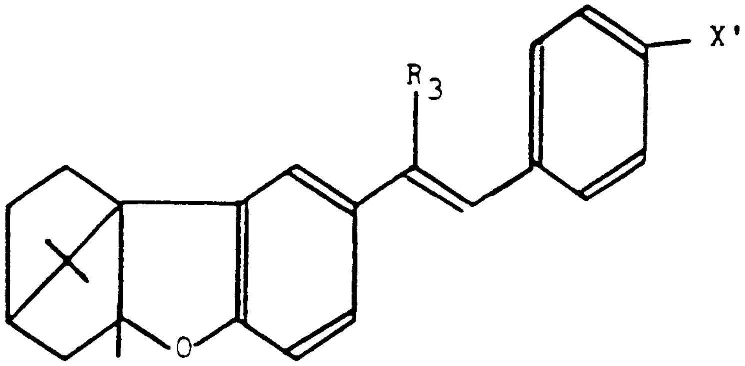

(20)

formule dans laquelle $R_3$ a la signification définie ci-dessus et X' représente un reste alkyl-thio carbamoyle avec un radical alkyle en $C_1$-$C_6$,avec une base ou un hydrure métallique.

**13.** Composition pharmaceutique, caractérisée par le fait qu'elle comporte, dans un support pharmaceutiquement acceptable, au moins un composé selon l'une des revendications 1 à 5.

**14.** Composition selon la revendication 13, caractérisée par le fait que la concentration en composé(s) selon l'une des revendications 1 à 5 est comprise entre 0,0005 et 2 % en poids et, de préférence, entre 0,002 et 1 % en poids.

**15.** Composition selon l'une des revendications 13 ou 14, caractérisée par le fait qu'elle se présente sous forme d'onguent, de gel, de crème, de pommade, de poudre, de teinture, de solution, de suspension, d'émulsion, de lotion, de spray, de timbre, de tampon imbibé, de comprimé , de gélule , de dragée , de sirop et de granulés.

**16.** Composition selon l'une des revendications 13 ou 14, caractérisée par le fait qu'elle se présente sous forme de collyre.

**17.** Composition selon l'une des revendications 13 à 16, caractérisée par le fait que le surport pharmaceutiquement acceptable de la composition renferme au moins un produit pris dans le groupe formé par l'eau, la gélatine, le lactose, l'amidon, le talc, la vaseline, la gomme arabique, les polyalcoylèneglycols, le stéarate de magnésium, les liants, les charges, les diluants, les solvants et les épaississants.

**18.** Utilisation de la composition selon la revendication 13 pour l'obtention d'un médicament destiné au traitement des affections dermatologiques liées à un désordre de la kératinisation portant sur la différenciation et sur la prolifération notamment pour le traitement des acnés vulgaires, comédoniennes, polymorphes, des acnés nodulo kystiques, conglobata, des acnés séniles, des acnés secondaires, telles que les acnés solaire, médicamenteuse, professionnelle ; pour le traitement d'autres types de trouble de la kératinisation, notamment les ichtyoses, les états ichtyosiformes, la maladie de Darier, les kératodermies palmoplantaires, les leucoplasies et états leucoplasiformes, le lichen ; pour le traitement d'autres affections dermatologiques liées à un trouble de la kératinisation avec une composante inflammatoire et/ou immuno-allergique, notamment toutes les formes de psoriasis qu'il soit cutané, muqueux ou unguéal, et le rhumatisme psoriasique ; pour le traitement de l'atopie cutanée, telle que l'eczema ou l'atopie respiratoire ; pour le traitement de toutes les proliférations dermiques ou épidermiques qu'elles soient bénignes ou malignes, ou qu'elles soient d'origine virale, telles que les verrues vulgaires, les verrues planes et l'épidermodysplasie verruciforme, ou qu'elles soient induites par les rayonnements ultra-violets, telles que les épithelioma baso et spino cellulaires ; pour le traitement d'autres désordres dermatologiques, tels que les maladies bulleuses et les maladies du collagène ; et pour les traitements d'ordre ophtalmologique, notamment les cornéopathies.

**19.** Utilisation pour l'obtention d'un médicament applicable par voie topique, selon la revendication 18, caractérisée par le fait que la concentration de la composition en substance(s) actives(s) est comprise entre 0,0005 % et 2 % en poids.

**20.** Utilisation pour l'obtention du médicament applicable par voie parentérale selon la revendication 18, caractérisée par le fait que la composition est sous forme de solution ou de suspension contenant de 0,01 mg à 1 mg de substance(s) active(s) par ml de composé(s) selon l'une des revendications 1 à 5.

**21.** Composition cosmétique, caractérisée par le fait qu'elle contient, dans un support cosmétiquement acceptable, au moins un composé selon l'une des revendications 1 à 5.

**22.** Composition selon la revendication 21, caractérisée par le fait que la concentration en composé(s) selon l'une des revendications 1 à 5 est comprise entre 0,0005 et 2 % en poids et, de préférence, entre 0,01 et 1 % en poids.

**23.** Composition selon l'une des revendications 21 ou 22, caractérisée par le fait qu'elle se présente sous forme de lotion, de gel, de crème, de mousse, de savon ou de shampooing.

**24.** Composition selon l'une des revendications 13 à 17 et 21 à 23, caractérisée par le fait qu'elle contient des additifs inertes ou cosmétiquement ou pharmaceutiquement actifs, pris dans le groupe formé par les agents hydratants, les agents anti-séborrhéiques, les agents anti-acnéiques, les antibiotiques, les agents favorisant la repousse des cheveux, les agents anti-inflammatoires, les caroténoïdes, les agents anti-psoriasiques, les agents de sapidité, les agents conservateurs, les agents stabilisants, les agents régulateurs d'humidité, les agents régulateurs de pH, les agents émulsionnants, les filtres UV-A et UV-B et les agents anti-oxydants.

**25.** Utilisation de la composition selon la revendication 21, pour l'obtention d'un médicament destiné à l'hygiène corporelle et capillaire et notamment pour le traitement des peaux à tendance acnéique, des peaux physiologiquement sèches, des séborrhées, de la chute des cheveux, pour la repousse des cheveux, pour le traitement et la prévention des effets néfastes du soleil, pour lutter contre l'aspect gras de la peau et des cheveux et pour le traitement du vieillissement de la peau.

**Claims**

**1.** Benzofuran compound, characterized in that it has the formula (I):

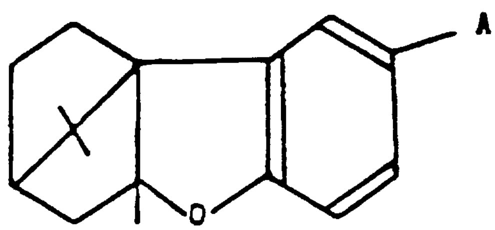

(I)

in which formula A represents:

- either a group (II):

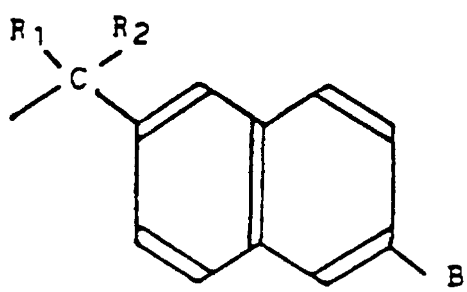

(II)

- or a group (III):

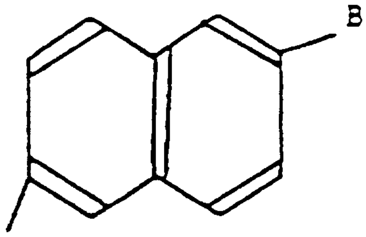

(III)

- or a group (IV):

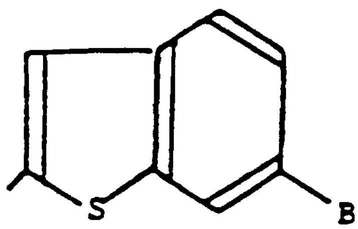

(IV)

- or a group (V):

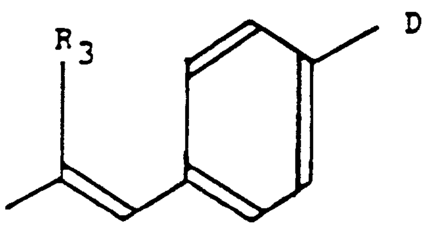

(V)

$R_1$ representing, in the group (II), a hydrogen atom, an OH radical, a $C_1$-$C_4$ alkoxy radical, a $C_1$-$C_4$ acyloxy radical or an $NH_2$ radical; $R_2$ representing, in the group (II), a hydrogen atom or a $C_1$-$C_4$ alkoxy radical; or else $R_1$ and $R_2$, taken together, form, in the group (II), an oxo, methano or hydroxyimino radical;

E representing, in the groups (II) to (IV):

- a CN radical;
- an oxazolinyl radical;
- a radical corresponding to the formula:

  $-CH_2OR_4$

  in which formula $R_4$ represents a hydrogen atom, a $C_1$-$C_6$ alkyl radical, a $C_2$-$C_6$ mono- or polyhydroxyalkyl radical, a cyclopentyl radical, a cyclohexyl radical or a tetrahydropyranyl radical;
- a radical corresponding to the formula:

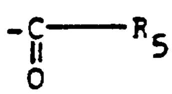

  in which $R_5$ represents:
  - a hydrogen atom;
  - a $C_1$-$C_6$ alkyl radical;

- a

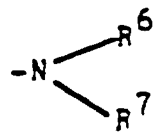

radical where $R^6$ and $R^7$ represent a hydrogen atom, a $C_1$-$C_6$ alkyl radical, a $C_3$-$C_6$ alkenyl radical, a cyclopentyl or cyclohexyl radical or also an optionally substituted aralkyl or aryl radical, it being possible for $R^6$ and $R^7$ to form a ring with the nitrogen atom to which they are attached, it additionally being possible for the

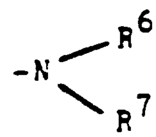

radical to be the residue of an amino acid or of an aminosugar

- A radical -O-$R_8$ where $R_8$ represents a hydrogen atom, a $C_1$-$C_{20}$ alkyl radical, or a $C_2$-$C_6$ mono- or polyhydroxyalkyl residue, it also being possible for the -O-$R_8$ group to be derived from a sugar;

- a radical D representing either an -SH residue or an -$S(O)_nR_9$ residue in which n is between 0 and 2 inclusive, $R_9$ representing:
  - a $C_1$-$C_6$ alkyl radical which is unsubstituted or substituted by one or a number of $C_2$-$C_6$ alkoxycarbonyl groups, one or a number of carboxyl groups, or one or a number of amino or dialkylamino groups or groups which can be a residue of an amino acid;
  - a $C_3$-$C_6$ alkenyl radical;
  - a $C_2$-$C_6$ mono- or polyhydroxyalkoyl radical;
  - and when n is equal to 1 or 2, a hydroxyl residue, a $C_1$-$C_6$ alkoxy residue or a residue

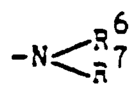

in which $R^6$ and $R^7$ can have the meanings defined above;

D having, in the group (V), the meaning given above and $R_3$ representing, in the group (V), a $C_1$-$C_6$ alkyl residue; and their salts and isomers.

**2.** Compound according to Claim 1, having a formula (I) in which A is a group (II), in which B is either $COOR_8$, with $R_8$ = H or a $C_1$-$C_{20}$ alkyl radical, or a radical $COR_5$ with
$R_5$ =

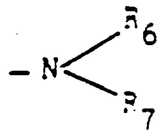
,

$R_6$ being H and $R_7$ being a $C_1$-$C_6$ alkyl radical.

**3.** Compound according to Claim 1, having a formula (I) in which A is a group (III) in which B is either $COR_5$ with $R_5$ =

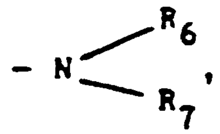

$R_6$ being a hydrogen and $R_7$ being a $C_1$-$C_6$ alkyl radical, or $COOR_8$ with $R_8$ = H or a $C_1$-$C_{20}$ alkyl radical.

4. Compound according to Claim 1, having a formula (I) in which A is a group (IV), where B represents $COOR_8$, $R_8$ being H or a $C_1$-$C_{20}$ alkyl radical.

5. Compound according to Claim 1, having a formula (I) in which A is a group (V) and D is an $-SCH_3$ residue.

6. Process for the preparation of a compound having a formula (I) according to Claim 1 in which A is the group (II), $R_1$ and $R_2$ together forming an oxo radical, characterized in that, in a first step, the product (1), consisting of a 6-alkoxycarbonyl-2-naphthalenecarboxylic acid, is converted to the corresponding chloride (2) by reacting with thionyl chloride; and, in a second step, the acid chloride (2) obtained is reacted with 2,3,4,4a-tetrahydro-4a,10,10-trimethyl-1H-3,9b-methanodibenzofuran (3), either directly by the Friedel-Crafts reaction or by reaction with the halogenated magnesium-containing derivative (4) prepared beforehand, it being possible for the benzofuran ester (5) obtained to be then saponified to give the corresponding acid (6).

7. Process according to Claim 6, characterized in that the benzofuran ester (5) or acid (6) obtained is used as starting material for the manufacture of another compound having a formula (I) in which A is the group (II).

8. Process for the preparation of a compound having a formula (I) according to Claim 1 in which A is the group (III), characterized in that a coupling reaction is carried out between a halogenated benzofuran derivative of formula:

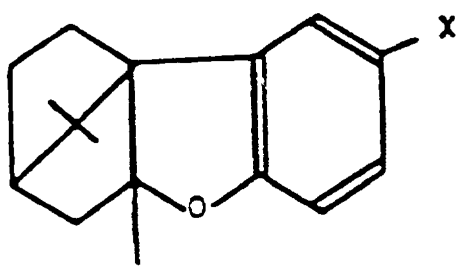

(10)

and a halogenated naphthalene derivative of formula:

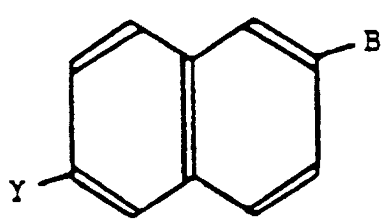

(11)

where B has the same meaning as in Claim 1 and X and Y represent a halogen atom, the halogenated derivative (10) being converted to a magnesium-, lithium- or zinc-containing derivative and then coupled with the halogenated naphthalene derivative (11) by using, as catalyst, a transition metal or one of its complexes.

9. Process for the preparation of a compound having a formula (I) according to Claim 1 in which A is a group (IV), characterized in that a benzofurancarboxylic acid derivative (12) of formula:

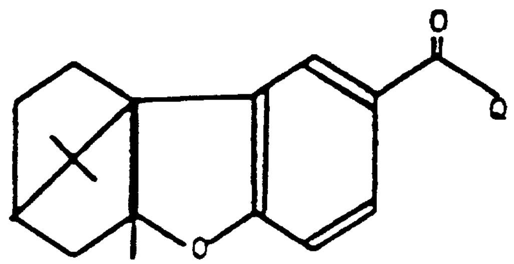

(12)

where Q = OH or Cl
is reacted with a thiol (13) of formula:

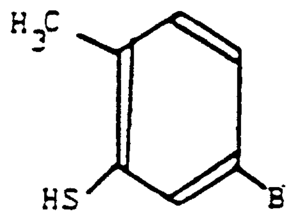

(13)

to obtain an intermediate product (14) of formula:

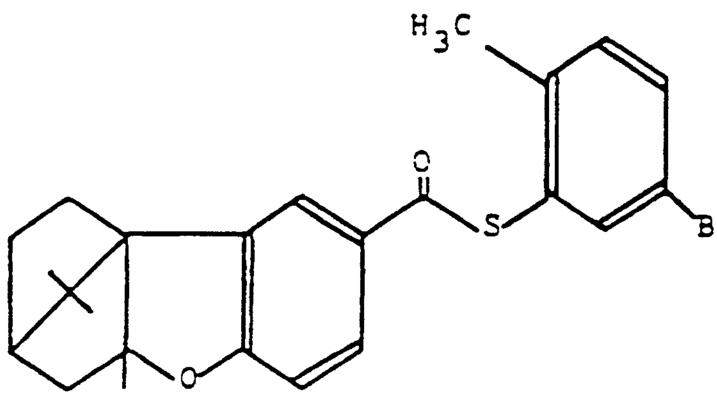

(14)

where B has the meaning given in Claim 1, and in that the product (14) is then brominated to obtain a bromomethylated derivative of formula:

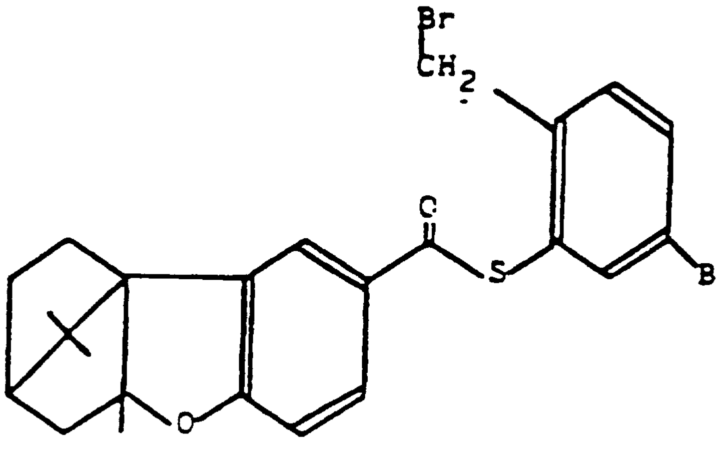

(15)

then reacted with a triarylphosphine and cyclized to obtain the product of formula:

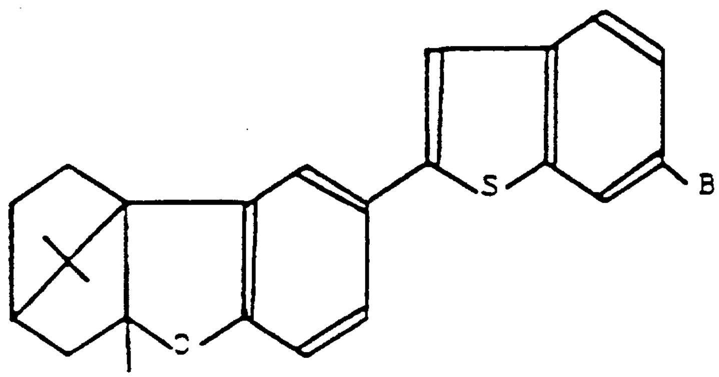

(16)

**10.** Process for the preparation of a compound having a formula (I) according to Claim 1 in which A is a group (V), characterized in that a benzofuran compound of formula:

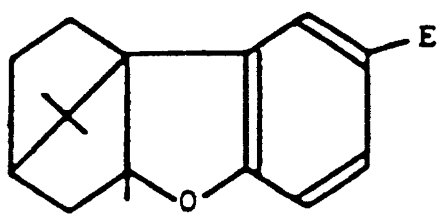

(17)

is reacted with a compound of formula:

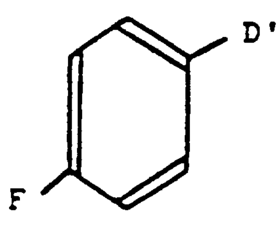

(18)

in which D' represents an $-SO_3^{\ominus}M^{\oplus}$, $-SO_2^{\ominus}M^{\oplus}$, alkylthio, alkylsulphinyl or alkylsulphonyl residue, in which the alkyl radical is $C_1$-$C_6$, E represents an

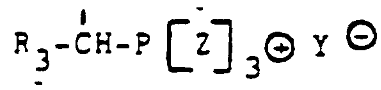

residue when F represents a formyl residue or else E represents a

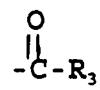

residue, when F represents a

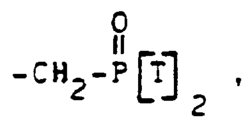

residue, Z representing an aryl residue, T representing a $C_1$-$C_6$ alkoxy residue, $Y^{\ominus}$ being an anion of an inorganic or organic acid and $M^{\oplus}$ being a cation of an alkali metal or alkaline-earth metal, $R_3$ having the meaning defined above.

**11.** Process for the preparation of a compound having a formula (I) according to Claim 1 in which A is a group (V), characterized in that a strong base is reacted with a compound of formula:

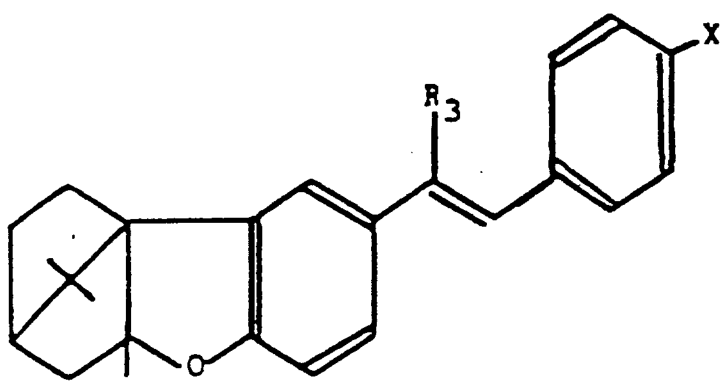

(19)

$R_3$ having, in the compound (19), the meaning defined above and X representing a halogen atom, and the product obtained is then subjected to the action of $SO_2$.

**12.** Process for the preparation of a compound having a formula (I) according to Claim 1 in which A is a group (V), characterized in that a compound of formula:

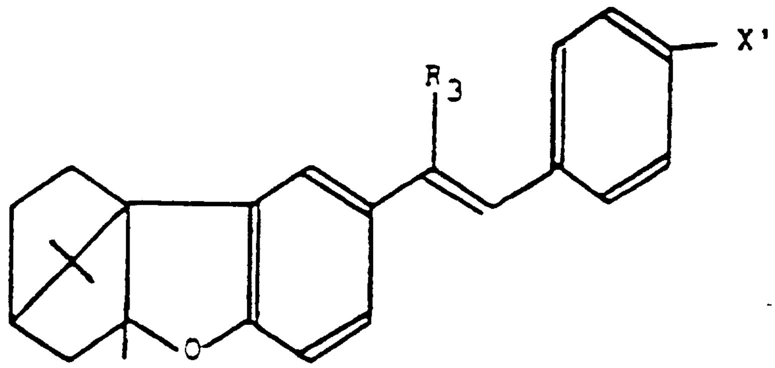

(20)

in which $R_3$ has the meaning defined above and X' represents an alkylthiocarbamoyl residue with a $C_1$-$C_6$ alkyl radical, is reacted with a base or a metal hydride.

**13.** Pharmaceutical composition, characterized in that it contains, in a pharmaceutically acceptable vehicle, at least one compound according to one of Claims 1 to 5.

**14.** Composition according to Claim 13, characterized in that the concentration of compound(s) according to one of Claims 1 to 5 is between 0.0005 and 2% by weight and preferably between 0.002 and 1% by weight.

**15.** Composition according to either of Claims 13 and 14, characterized in that it is provided in the ointment, gel, cream, salve, powder, tincture, solution, suspension, emulsion, lotion, spray, stamp, impregnated pad, tablet, gelatin capsule, dragée, syrup and granules forms.

**16.** Composition according to either of Claims 13 and 14, characterized in that it is provided in the eyewash form.

**17.** Composition according to one of Claims 13 to 16, characterized in that the pharmaceutically acceptable vehicle for the composition contains at least one product taken from the group formed by water, gelatin, lactose, starch, talc, petroleum jelly, gum arabic, polyalkylene glycols, magnesium stearate, binding agents, fillers, diluents, solvents and thickening agents.

**18.** Use of the composition according to Claim 13 for producing a medicament intended for the treatment of dermatological conditions linked to a disorder of keratinization involving differentiation and proliferation, in particular for the treatment of acne vulgaris, comedonic or polymorphic acne, nodulocystic acne, acne conglobata, senile acne and secondary acnes such as solar, drug or occupational acne; for the treatment of other types of disorders of keratinisation, especially ichthyoses, ichthyosiform conditions, Darier's disease, palmoplantar keratoderma, leucoplakia and leucoplakiform conditions or lichen; for the treatment of other dermatological conditions linked to a disorder of keratinisation with an inflammatory and/or immunoallergic component, in particular all forms of psoriasis, either cutaneous, mucosal or ungual, and psoriatic rheumatism; for the treatment of cutaneous atopy such as eczema or respiratory atopy; for the treatment of all dermal or epidermal proliferations, whether they are benign or malignant or whether they are of viral origin, such as common warts, flat warts and epidermodysplasia verruciformis, or whether they are induced by ultraviolet radiation, such as basal cell and prickle cell epithelioma; for the treatment of other dermatological disorders, such as vesicular diseases and collagen diseases; and for treating ophthalmological disorders, in particular corneopathies.

**19.** Use in producing a topically applicable medicament according to Claim 18, characterized in that the concentration in the composition of active substance(s) is between 0.0005% and 2% by weight.

**20.** Use in producing a parenterally applicable medicament according to Claim 18, characterized in that the composition is in the form of a solution or suspension containing from 0.01 mg to 1 mg of active substance(s) per ml of compound(s) according to one of Claims 1 to 5.

**21.** Cosmetic composition, characterized in that it contains, in a cosmetically acceptable vehicle, at least one compound according to one of Claims 1 to 5.

**22.** Composition according to Claim 21, characterized in that the concentration of compound(s) according to one of Claims 1 to 5 is between 0.0005 and 2% by weight and preferably between 0.01 and 1% by weight.

**23.** Composition according to either of Claims 21 and 22, characterized in that it is provided in the lotion, gel, cream, foam, soap or shampoo form.

**24.** Composition according to one of Claims 13 to 17 and 21 to 23, characterized in that it contains inert additives or cosmetically or pharmaceutically active additives taken from the group formed by hydrating agents, anti-seborrhoeic agents, anti-acne agents, antibiotics, agents promoting hair regrowth, antiinflammatory agents, carotenoids, anti-psoriatic agents, flavouring agents, preserving agents, stabilizing agents, moisture-regulating agents, pH regulating agents, emulsifying agents, UV-A and UV-B screening agents and anti-oxidizing agents.

**25.** Use of the composition according to Claim 21, for producing a medicament intended for body and hair hygiene and in particular for the treatment of skins having a tendency towards acne, physiologically dry skins, seborrhoeas, hair loss, for hair regrowth, for the treatment and prevention of the harmful effects of the sun, for combating the greasy appearance of the skin and hair and for the treatment of aging of the skin.

**Patentansprüche**

**1.** Benzofuranverbindung, gekennzeichnet durch die allgemeine Formel (I):

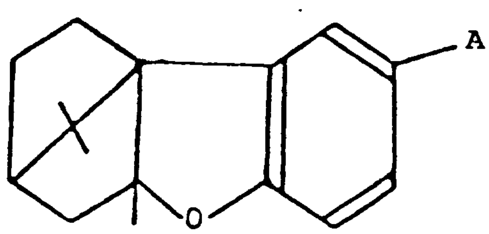

(I)

worin A
- für eine Gruppe (II):

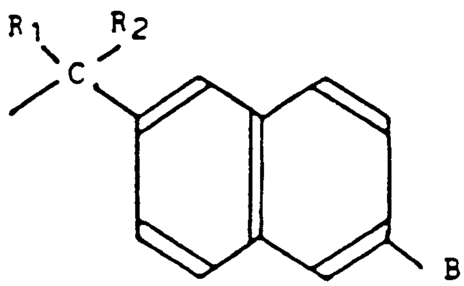

(II)

- oder für eine Gruppe (III):

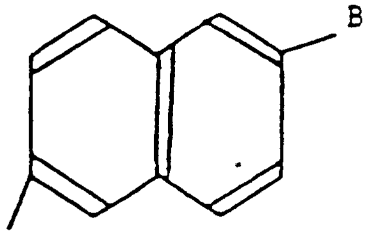

(III)

- oder für eine Gruppe (IV):

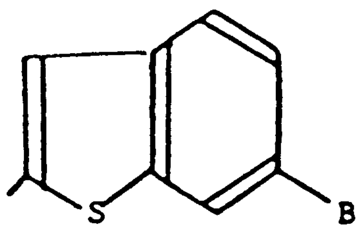

(IV)

- oder für eine Gruppe (V):

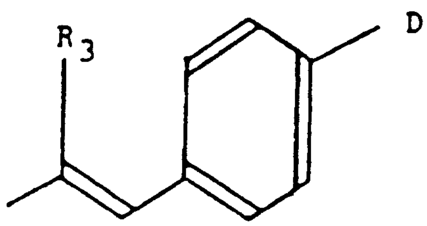

(V)

steht, wobei
in der Gruppe (II) $R_1$ für ein Wasserstoffatom, einen OH-Rest, eine $C_1$-$C_4$-Alkoxygruppe, eine $C_1$-$C_4$-Acyloxygruppe oder für eine $NH_2$-Gruppe steht; $R_2$ für ein Wasserstoffatom oder für einen $C_1$-$C_4$-Alkoxyrest steht; oder $R_1$ und $R_2$ zusammen einen Oxo-, Methano- oder Hydroxyiminomorest bilden;
in den Gruppen (II) bis (IV) B steht für:

- einen CN-Rest;
- einen Oxazolinyl-Rest;
- einen Rest der Formel:

  $-CH_2OR_4$

  worin $R_4$ für ein Wasserstoffatom, einen $C_1$-$C_6$-Alkylrest, einen Mono- oder Polyhydroxy-$C_2$-$C_6$-Alkylrest, einen Cyclopentylrest, einen Cyclohexylrest oder einen Tetrahydropyranylrest steht;
- einen Rest der Formel:

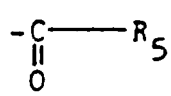

  worin $R_5$ bedeutet:
  * ein Wasserstoffatom;
  * einen $C_1$-$C_6$-Alkylrest;

* einen Rest der Formel

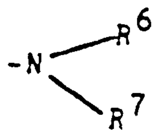

worin $R^6$ und $R^7$ jeweils ein Wasserstoffatom, einen $C_1$-$C_6$ Alkylrest, einen $C_3$-$C_6$ Alkenylrest, einen Cyclopentyl- oder Cyclohexylrest oder einen Aralkyl- oder Arylrest bedeuten, der gegebenenfalls substituiert sein kann, wobei $R^6$ und $R^7$ mit dem Stickstoffatom an das sie gebunden sind, einen Ring bilden können, wobei der Rest

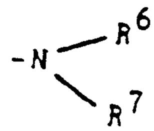

unter anderem der Rest einer Aminosäure oder eines Aminozuckers sein kann;

* einen Rest -O-$R_8$, worin $R_8$ für ein Wasserstoffatom, einen $C_1$-$C_{20}$-Alkylrest, einen Mono- oder Polyhydroxy-$C_2$-$C_6$-Alkylrest bedeutet, wobei die Gruppe -O-$R_8$ ebenfalls von einem Zucker abgeleitet sein kann;

- einen Rest D, der entweder für einen HS-Rest oder für einen Rest der Formel -S(O)$_n$$R_9$ steht, worin n einen Wert von einschließlich 0 bis einschließlich 2 einnehmen kann und $R_9$ bedeutet:
  * einen $C_1$-$C_6$-Alkylrest, der gegebenenfalls substituiert ist mit einer oder mehreren $C_2$-$C_6$-Alkoxycarbonylgruppen, einer oder mehreren Carboxylgruppen, einer oder mehreren Amino- oder Dialkylaminogruppen, oder für den Rest einer Aminosäure stehen kann;
  * einen $C_3$-$C_6$-Alkenylrest;
  * einen Mono- oder Polyhydroxy-$C_2$-$C_6$-Alkoylrest;
  * und wenn n für 1 oder 2 steht, für einen Hydroxyrest, einen $C_1$-$C_6$-Alkoxyrest oder einen Rest der Formel

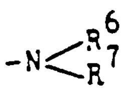

worin $R^6$ und $R^7$ die oben angegebenen Bedeutungen besitzen können;

in der Gruppe (V) D die oben angegebenen Bedeutungen besitzt und $R_3$ für einen $C_1$-$C_6$-Alkylrest steht; sowie die Salze und Isomere davon.

**2.** Verbindung nach Anspruch 1 der Formel (I), worin A für eine Gruppe (II) steht, worin B für $COOR_8$ steht, wobei $R_8$ ein Wasserstoffatom oder einen $C_1$-$C_{20}$-Alkylrest oder einen Rest $COR_5$ bedeutet, wobei $R_5$ für

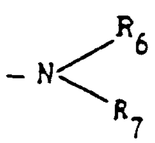

steht und $R_6$ ein Wasserstoffatom und $R_7$ einen $C_1$-$C_6$-Alkylrest bedeutet.

**3.** Verbindung nach Anspruch 1 der Formel (I), worin A für eine Gruppe (III) steht, wobei B für $COR_5$ steht, worin $R_5$ die Formel

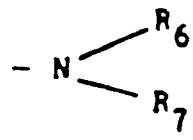

besitzt und $R_6$ ein Wasserstoffatom und $R_7$ einen $C_1$-$C_6$-Alkylrest bedeutet; oder für $COOR_8$ steht, worin $R_8$ ein Wasserstoffatom oder einen $C_1$-$C_{20}$-Alkylrest bedeutet.

**4.** Verbindung nach Anspruch 1 der Formel (I), worin A für eine Gruppe (IV) steht, wobei B für $COOR_8$ steht, worin $R_8$ ein Wasserstoffatom oder einen $C_1$-$C_{20}$-Alkylrest bedeutet.

**5.** Verbindung nach Anspruch 1 der Formel (I), worin A für eine Gruppe (V) steht und D für einen Rest $-SCH_3$ steht.

**6.** Verfahren zur Herstellung einer Verbindung der Formel (I) gemäß Anspruch 1, worin A für eine Gruppe (II) steht und $R_1$ und $R_2$ zusammen einen Oxorest bilden, dadurch gekennzeichnet, daß man zunächst das von einer Alkoxycarbonyl-6-naphthalin-2-carbonsäure gebildete Produkt (1) unter der Einwirkung von Thionylchlorid in das entsprechende Chlorid (2) überführt; und in einem zweiten Schritt das erhaltene Säurechlorid (2) mit 2,3,4,4a-Tetrahydro-4a,10,10-Trimethyl-1H-3,9b-methanodibenzofuran (3) entweder direkt mit Hilfe der Friedel-Crafts-Reaktion oder durch Reaktion mit einem zuvor hergestellten Magnesiumhalogenderivat (4) umsetzt, wobei der erhaltene Benzofuranester (5) anschließend unter Bildung der entsprechenden Säure (6) verseift werden kann.

**7.** Verfahren nach Anspruch 6, dadurch gekennzeichnet, daß man den erhaltenen Benzofuran-Ester (5) oder die Säure (6) als Ausgangsprodukt für die Herstellung einer anderen Verbindung der Formel (I) verwendet, worin A für die Gruppe (II) steht.

**8.** Verfahren zur Herstellung einer Verbindung der Formel (I) gemäß Anspruch 1, worin A für die Gruppe (III) steht, dadurch gekennzeichnet, daß man eine Kopplungsreaktion durchführt zwischen einem halogenierten Benzofuranderivat der Formel:

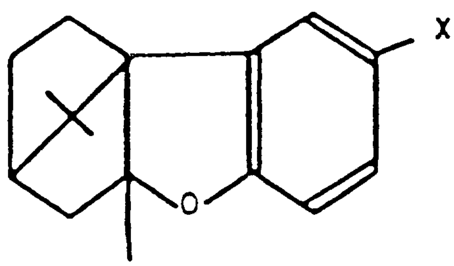

(10)

und einem Halogenderivat des Naphthalins der Formel:

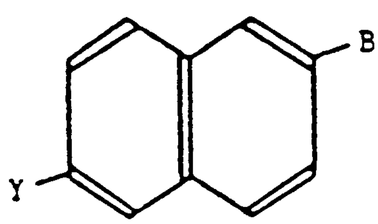

(11)

worin B wie in Anspruch 1 definiert ist und X und Y für ein Halogenatom stehen, wobei das Halogenderivat (10) in die Magnesium-, Lithium- oder Zinkform überführt wird, anschließend mit dem Naphthalin-Halogenderivat (11) gekoppelt wird, indem man als Katalysator ein Übergangsmetall oder einen Komplex davon verwendet.

**9.** Verfahren zur Herstellung einer Verbindung der Formel (I) gemäß Anspruch 1, worin A für die Gruppe (IV) steht, dadurch gekennzeichnet, daß man ein Benzofurancarbonsäurederivat (12) der Formel:

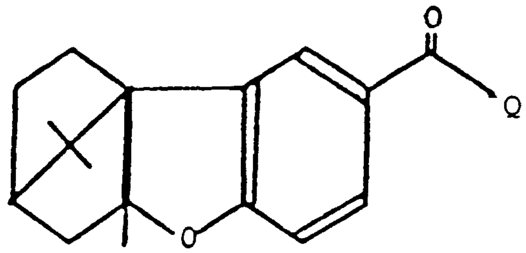

(12)

worin Q für OH oder Cl steht,
mit einem Thiol (13) der Formel:

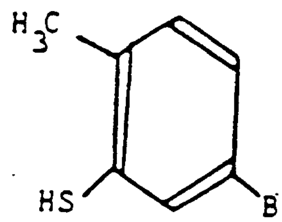

(13)

reagieren läßt, wobei man ein Zwischenprodukt (14) der Formel:

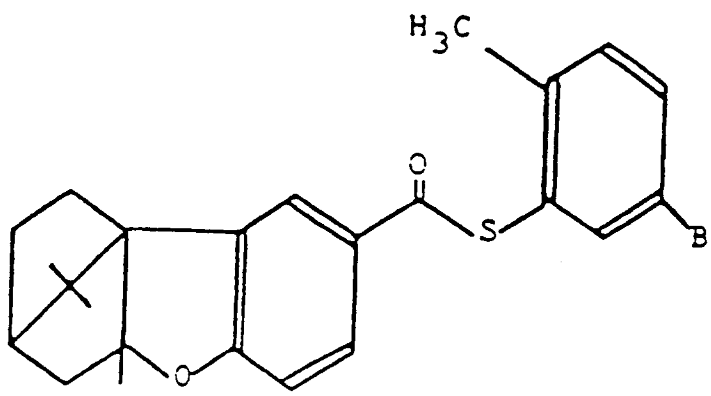

(14)

erhält, worin B wie in Anspruch 1 definiert ist, man das Produkt (14) anschließend einer Bromierung unterzieht, um das Brommethylderivat der Formel:

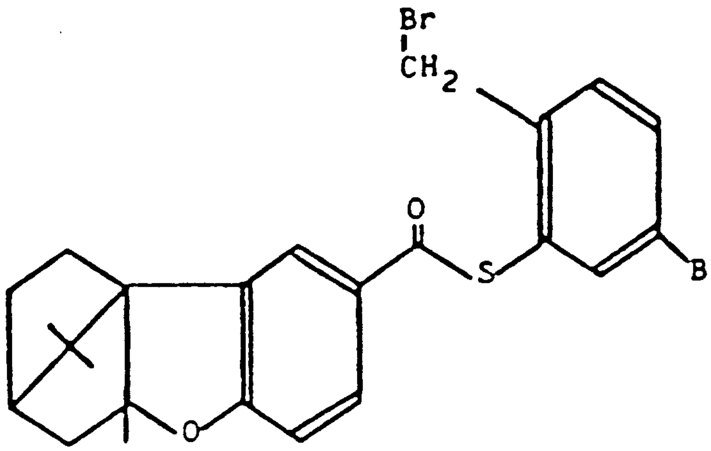

(15)

zu erhalten, man anschließend eine Reaktion mit einem Triarylphosphin und eine Cyclisierung durchführt, um das Produkt der Formel:

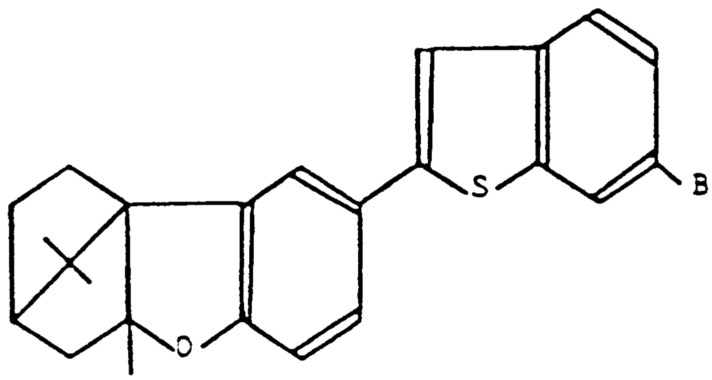

(16)

zu erhalten.

**10.** Verfahren zur Herstellung einer Verbindung der Formel (I) nach Anspruch 1, worin A für eine Gruppe (V) steht, dadurch gekennzeichnet, daß man eine Benzofuranverbindung der Formel:

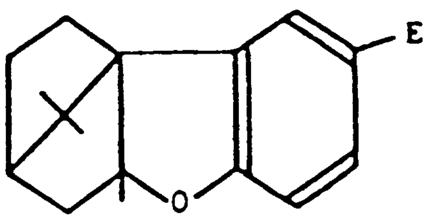

(17)

mit einer Verbindung der Formel:

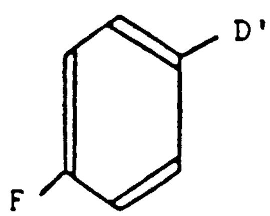

(18)

umsetzt, worin D' für $-SO_3^{\ominus}M^{\oplus}$, $-SO_2^{\ominus}M^{\oplus}$, Alkylthio, Alkylsulfinyl oder Alkylsulfonyl steht, worin der

Alkylrest ein $C_1$-$C_6$-Alkylrest ist, E für einen Rest

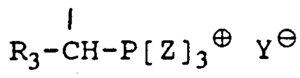

steht, wenn F für einen Formylrest steht, oder worin E für einen Rest

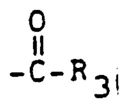

steht, wenn F für einen Rest

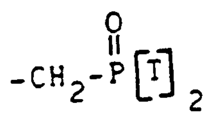

steht, Z für einen Arylrest steht,
T für einen $C_1$-$C_6$-Alkoxyrest steht, $Y^{\ominus}$ für ein Anion einer mineralischen oder organischen Säure steht und $M^{\oplus}$ für ein Kation eines Alkali- oder Erdalkalimetalls steht und $R_3$ wie oben angegeben definiert ist.

**11.** Verfahren zur Herstellung einer Verbindung der Formel (I) nach Anspruch 1, worin A für eine Gruppe (V) steht, dadurch gekennzeichnet, daß man eine starke Base auf eine Verbindung der Formel:

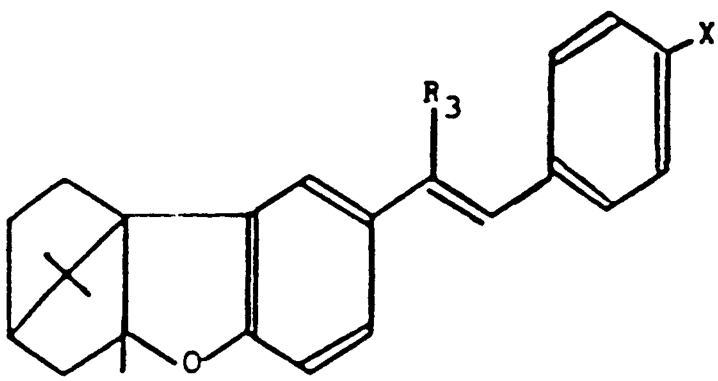

(19)

einwirken läßt, worin $R_3$ in der Verbindung (19) wie oben angegeben definiert ist und X für ein Halogenatom steht, und man anschließend auf das erhaltene Produkt $SO_2$ einwirken läßt.

**12.** Verfahren zur Herstellung einer Verbindung der Formel (I) nach Anspruch 1, worin A für eine Gruppe (V) steht, dadurch gekennzeichnet, daß man eine Verbindung der Formel:

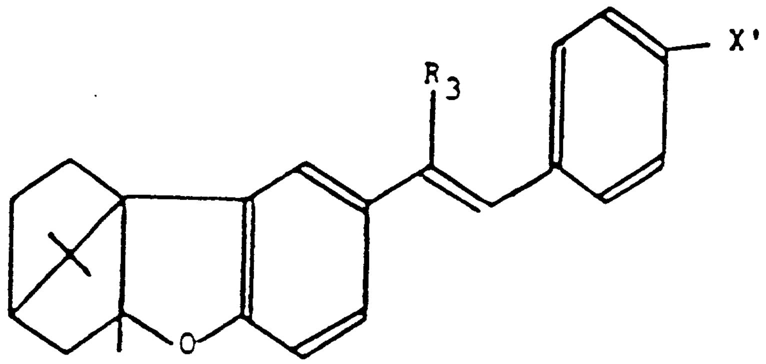

(20)

worin $R_3$ wie oben angegeben definiert ist und X' für einen Alkylthiocarbamoylrest steht, wobei der Alkylrest ein $C_1$-$C_6$-Alkylrest ist, mit einer Base oder einem Metallhydrid reagieren läßt.

**13.** Pharmazeutische Zusammensetzung, dadurch gekennzeichnet, daß sie in einem pharmazeutisch verträglichen Träger wenigstens eine Verbindung nach einem der Ansprüche 1 bis 5 umfaßt.

**14.** Zusammensetzung nach Anspruch 13, dadurch gekennzeichnet, daß die Konzentration der Verbindung(en) gemäß einem der Ansprüche 1 bis 5 im Bereich von 0,0005 bis 2 Gew.-%, vorzugsweise von 0,002 bis 1 Gew.-%, liegt.

**15.** Zusammensetzung nach einem der Ansprüche 13 oder 14, dadurch gekennzeichnet, daß sie in Form einer Salbe, eines Gels, einer Creme, einer Pommade, eines Pulvers, einer Tinktur, einer Lösung, einer Suspension, einer Emulsion, einer Lotion, eines Sprays, eines Stempels, eines getränkten Tupfers, einer Tablette, einer Kapsel, eines Dragees, eines Sirups oder als Granulat vorliegt.

**16.** Zusammensetung nach einem der Ansprüche 13 oder 14, dadurch gekennzeichnet, daß sie in Form eines Collyriums vorliegt.

**17.** Zusammensetzung nach einem der Ansprüche 13 bis 16, dadurch gekennzeichnet, daß der pharmazeutisch verträgliche dadurch gekennzeichnet, daß der pharmazeutisch verträgliche Träger der Zusammensetzung wenigstens ein Produkt umfaßt, das ausgewählt ist unter Wasser, Gelatine, Lactose, Stärke, Talkum, Vaseline, Gummi Arabikum, Polyalkylenglykolen, Magnesiumsterat, Bindemittel, Füllstoffe, Verdünnungsmittel, Lösungsmittel und Verdickungsmittel.

**18.** Verwendung der Zusammensetzung gemäß Anspruch 13 zur Herstellung eines Medikaments für die Behandlung dermatologischer Erkrankungen, die mit einer Störung der Keratinisation bei der Differenzierung und Proliferation verbunden sind, insbesondere zur Behandlung von Akne vulgaris, comedonica und polymorpher Akne, nodulärer zystischer Akne, Akne conglobata, seniler Akne, Sekundärakne, wie Sonnen-, Medikamenten- oder berufsbedingter Akne; zur Behandlung anderer Arten von Störungen der Keratinisation, insbesondere Ichtyosen, ichtyosiformen Zuständen, der Darier-Krankheit, von Palmoplantarkeratosen, Leukoplasien und leukoplasiformen Zuständen, sowie von Lichen; zur Behandlung anderer dermatologischer Affektionen, die mit einer Störung der Keratinisation verbunden sind und eine inflammatorische und/oder immunoallergische Komponente besitzen, insbesondere von allen Psoriasisformen der Haut, der Schleimhaut oder der Nägel, sowie von psoriatischem Rheumatismus; zur Behandlung von kutaner Atopie, wie Ekzemen oder respiratorischer Atopie; zur Behandlung sämtlicher Formen benigner oder maligner dermaler oder epidermaler Prolife-rationen, viralen Ursprungs, wie Verruca vulgaris, Verruca plana und verruciforme Epidermoplasie, oder durch ultra-violette Strahlung induzierte Formen, wie Epithelioma baso-cellulare oder spinocellulare; zur Behandlung anderer dermatologischer Störungen, wie bullösen Erkrankungen und kollagenalen Erkrankungen; sowie zur ophthalmologischen Behandlung, insbesondere zur Behandlung von Korneopathien.

**19.** Verwendung zur Herstellung eines topisch applizierbaren Medikaments gemäß Anspruch 18, dadurch gekennzeichnet, daß die Konzentration der Zusammensetzung in bezug auf die aktive(n) Substanz(en) im Bereich von 0,0005 Gew.-% bis 2 Gew.-% liegt.

**20.** Verwendung zur Herstellung eines parenteralen applizierbaren Medikaments nach Anspruch 18, dadurch gekennzeichnet, daß die Zusammensetzung in Form einer Lösung oder einer Suspension vorliegt, die 0,01 mg bis 1 mg aktive Substanz(en) pro ml in Form der Verbindung(en) gemäß einem der Ansprüche 1 bis 5 enthält.

**21.** Kosmetische Zusammensetzung, dadurch gekennzeichnet, daß sie in einem kosmetisch verträglichen Träger wenigstens eine Verbindung nach einem der Ansprüche 1 bis 5 enthält.

**22.** Zusammensetzung nach Anspruch 21, dadurch gekennzeichnet, daß die Konzentration der Verbindung(en) nach einem der Ansprüche 1 bis 5 im Bereich von 0,0005 bis 2 Gew.-% und vorzugsweise im Bereich von 0,01 bis 1 Gew.-% liegt.

**23.** Zusammensetzung nach einem der Ansprüche 21 oder 22, dadurch gekennzeichnet, daß sie in Form einer Lotion, als Gel, als Creme, als Schaum, als Seife oder als Shampoo vorliegt.

**24.** Zusammensetzung nach einem der Ansprüche 13 bis 17 und 21 bis 23, dadurch gekennzeichnet, daß sie inerte oder kosmetisch oder pharmazeutisch aktive Zusätze enthält, die ausgewählt sind unter Feuchthaltemitteln, Antiseborrhoe-Mitteln, Antiakne-Mitteln, Antibiotika, Mitteln zur Förderung des Nachwachsens der Haare, antiinflammatorischen Mitteln, Carotinoiden, Antipsoriasis-Mitteln, Geschmacksmitteln, Konservierungsmitteln, Stabilisierungsmitteln, feuchtigkeitsregulierenden Mitteln, pH-regulierenden Mitteln, Emulgiermitteln, UV-A- und UV-B-Filtern und Antioxidantien.

**25.** Verwendung der Zusammensetzung nach Anspruch 21 zur Herstellung eines Medikamts zur Körper- und Haar-Hygiene und insbesondere zur Behandlung von zur Akne neigender Haut, von physiologisch trockener Haut, von Seborrhoe, von Haarausfall, zur Förderung des Nachwachsens der Haare, zur Behandlung von und zur Vorbeugung vor schädigenden Einflüssen der Sonne, zur Bekämpfung eines fettigen Aussehens von Haut und Haar und zur Behandlung der Hautalterung.